(19) Europäisches Patentamt
European Patent Office
Office européen des brevets

(11) Publication number: **0 271 041 A2**

(12) # EUROPEAN PATENT APPLICATION

(21) Application number: **87118060.0**

(22) Date of filing: **07.12.87**

(51) Int. Cl.⁴: **C07K 7/08**, C07K 7/06, A61K 37/24

A request for correction of the description has been filed pursuant to Rule 88 EPC. A decision on the request will be taken during the proceedings before the Examining Division (Guidelines for Examination in the EPO, A-V, 2.2).

(30) Priority: **10.12.86 US 940472**
**30.10.87 US 112606**

(43) Date of publication of application:
**15.06.88 Bulletin 88/24**

(84) Designated Contracting States:
**BE CH DE ES FR GB GR IT LI NL SE**

(71) Applicant: **ABBOTT LABORATORIES**
**Abbott Park Illinois 60064(US)**

(72) Inventor: **Holleman, William Homer**
**15460 W. Timber Lane**
**Libertyville Illinois 60048(US)**
Inventor: **Rockway, Todd Warren**
**W38 Oakdale Lane**
**Mundelein Illinois 60060(US)**
Inventor: **Devine, Edward Michael, Jr.**
**15244 Pinewood**
**Libertyville Illinois 60048(US)**
Inventor: **Connolly, Peter John**
**26 Whitebirch Road**
**Morristown New Jersey 07960(US)**

(74) Representative: **Modiano, Guido et al**
**MODIANO, JOSIF, PISANTY & STAUB**
**Modiano & Associati Via Meravigli, 16**
**I-20123 Milan(IT)**

(54) **Atrial peptide derivatives.**

(57) Atrial peptides comprising the amino acid sequence as follows:

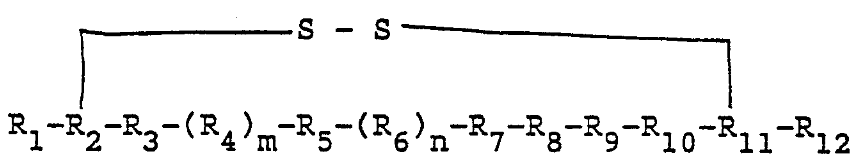

wherein $R_1$ is absent, hydrogen, 5-aminopentanoic acid, 8-aminooctanoic acid, 7-aminoheptanoic acid, acetyl, Ser, SerSer, D-Arg, Arg, ArgSerSer or ArgArgSerSer; $R_2$ is Cys, beta-mercaptopropionic acid or Pen; $R_3$ is Phe, Cha, L-Tic, D-Tic or Nal; $R_4$ is Gly, Pro, D-Ala or Ala; $R_5$ is Gly, 6-aminocaproic acid, meta-aminomethylbenzoic acid, Pro, Ala or D-Ala; $R_6$ is Arg, D-Arg, Orn, Lys or Gly; $R_7$ is Ile, Leu, PropargylGly or Gly; $R_8$ is Asp or Gly; $R_9$ is D-Arg, Arg, Orn, Lys or Gly; $R_{10}$ is Ile; $R_{11}$ is Cys or Pen; $R_{12}$ is OH, $NH_2$, ChaArg, ProArg, PheArg, GlyArg, Arg,PheLys, (8-aminooctanoyl)Arg, AsnSer, SerPheArg or AsnSerPheArg; m is 0 or 1 and n is 0 or 1; or pharmaceutically acceptable salts, esters or amides thereof.

EP 0 271 041 A2

# ATRIAL PEPTIDE DERIVATIVES

The present invention relates to derivatives of atrial natriuretic polypeptides useful for the treatment of hypertension.

## Background Art:

Recently, alpha-human atrial natriuretic polypeptide (alpha-hANP) has been isolated from human atrium and identified as a polypeptide consisting of 28 amino acids having the following formula:

```
Ser Leu Arg Arg Arg Ser Ser Cys Phe Gly Gly Arg Ile Asp-
                            |
                            S ------ S
                                     |
Arg Ile Gly Ala Gln Ser Gly Leu Gly Cys Asn Ser Phe Arg-
Tyr
```

It has been reported that the diuretic action of the alpha-hANP was about 1000 times as strong as that of furosemide which is used as a hypotensive diuretic.

Others have found alpha-hANP increases urinary sodium and urinary output.

However, alpha-hANP or derivatives thereof have not been commercially available and research on ANP derivatives which are chemically and enzymatically stable, have long duration and stronger action than alpha-hANP has been actively pursued.

Heretofore, hypertension has been classified into hypertension having clear factor disease and one having obscure factor disease. The former is classified as secondary hypertension and involves mainly those caused by kidney disease, endocrine disease, toxemia of pregnancy, arterial embolism, central nervous system disease and the like. Alternatively, the later hypertension having obscure factor disease is classified as essential hypertension, in which 80-90% of hypertension patients have been classified. Though the conditions of the secondary hypertension can be improved by treating the factor disease, the treatment of conditions of the essential hypertension having obscure factor disease have been effected by, for example, the administration of a hypotensive diuretic or vasodilator as symptomatic therapy. Recently, it has been suggested that the above alpha-hANP is one of the materials which is related to the cause of heart disease or essential hypertension and the research on essential hypertension concerning alpha-hANP has drawn attention.

## Brief Description of the Invention

In accordance with the present invention, novel peptides are provided which exhibit useful hypotensive,

natriuretic, diuretic and vasorelaxant activity. These biologically active peptides have the following amino acid sequence:

S - S (disulfide bridge between $R_2$ and $R_{11}$)

$$R_1-R_2-R_3-(R_4)_m-R_5-(R_6)_n-R_7-R_8-R_9-R_{10}-R_{11}-R_{12}$$

(Formula I)

wherein $R_1$ is absent, hydrogen, acetyl, Ser, SerSer, D-Arg, Arg, Apa, Ahepa, Aoa, ArgSerSer or ArgArgSerSer; $R_2$ is Cys, Mpa or Pen; $R_3$ is Phe Cha, L-Tic, D-Tic or Nal; $R_4$ is Gly, Pro, D-Ala or Ala; $R_5$ is Gly, Pro, Aca, mAMBA, Ala or D-Ala; $R_6$ is Arg, D-Arg, Lys, or Gly; $R_7$ is Ile, Leu, PrGly or Gly; $R_8$ is Asp or Gly; $R_9$ is D-Arg, Arg, Lys or Gly; $R_{10}$ is Ile; $R_{11}$ is Cys or Pen; $R_{12}$ is OH, $NH_2$, ChaArg, Arg, ProArg, GlyArg, PheArg PheLys, AoaArg, AsnSer, SerPheArg or AsnSerPheArg; m is 0 or 1 and n is 0 or 1; or pharmaceutically acceptable salts, esters or amides thereof.

In the above peptide structure, the amino acid components are designated by conventional abbreviations as follows:

| Amino Acid | Abbreviated Designation |
|---|---|
| Alanine | Ala |
| 6-Aminocaproic Acid | Aca |
| 7-Aminoheptanoic Acid | Ahepa |
| 8-Aminooctanoic Acid | Aoa |
| 5-Aminopentanoic Acid | Apa |
| Arginine | Arg |
| Asparagine | Asn |
| Aspartic Acid | Asp |

| | |
|---|---|
| Asparagine and/or Aspartic Acid | Asx |
| Cyclohexylalanine | Cha |
| Cysteine | Cys |
| Glutamine | Gln |
| Glycine | Gly |
| Isoleucine | Ile |
| Leucine | Leu |
| Lysine | Lys |
| m-Aminomethylbenzoic Acid | mAMBA |
| Methionine | Met |
| beta-Mercaptopropionic Acid | Mpa |
| Naphthylalanine | Nal |
| Ornithine | Orn |
| Penicillamine | Pen |
| Phenylalanine | Phe |
| Proline | Pro |
| Propargylglycine | PrGly |
| Serine | Ser |
| Tetrahydroisoquinoline 3-carboxylic Acid | Tic |
| Threonine | Thr |
| Tyrosine | Tyr |

Unless expressly stated otherwise, the optically active amino acids always have the L-configuration.

The term "aryl" as used herein means phenyl, benzyl or naphthyl groups.

Examples of pharmaceutically acceptable, non-toxic acid addition salts are salts formed with inorganic acids such as hydrochloric acid, hydrobromic acid, phosphoric acid, sulfuric acid and perchloric acid or with organic acids such as acetic acid, oxalic acid, maleic acid, malic acid, tartaric acid, citric acid, succinic acid or malonic acid. Other pharmaceutically acceptable salts include inorganic nitrate, sulfate, acetate, malate, formate, lactate, tartrate, succinate, citrate, p-toluenesulfonate, and the like or metal salts

such as sodium, potassium or calcium salts or ammonium or amino salts such as ammonium, triethylamine salts, and the like and they may be prepared according to conventional methods.

Examples of pharmaceutically acceptable, non-toxic esters of the compound of formula I include $C_1$ to $C_6$ alkyl esters wherein the alkyl group is straight or branched chain. Acceptable esters also include $C_5$ to $C_7$ cycloalkyl esters. $C_1$ to $C_4$ alkyl esters are preferred. Esters of the compound of formula I may be prepared according to conventional methods.

Examples of pharmaceutically acceptable, non-toxic amides of the compound of formula I include amides derived from ammonia, primary $C_1$ to $C_6$ alkyl amines and secondary $C_1$ to $C_6$ dialkyl amines wherein the alkyl groups are straight or branched chain. In the case of secondary amines the amine may also be in the form of a 5 or 6 membered heterocycle containing one nitrogen atom. Amides derived from ammonia, $C_1$ to $C_3$ alkyl primary amines and $C_1$ to $C_2$ dialkyl secondary amines are preferred. Amides of the compound of formula I may be prepared according to conventional methods.

The daily dosage of the compounds of the present invention may be suitably defined according to the conditions of the patient by those skilled in the art but generally may be administered in an amount of about 0.2-250 mg/kg body weight. Dosage unit compositions may contain such amounts of submultiples thereof to make up the daily dose.

The amount of active ingredient that may be combined with carrier materials to produce a single dosage form will vary depending upon the host treated and the particular mode of administration.

It will be understood, however, that the specific dose level for any particular patient will depend upon a variety of factors including the activity of the specific compound employed, the age, body weight, general health, sex, diet, time of administration, route of administration, rate of excretion, drug combination, and the severity of the particular disease undergoing therapy.

The compounds of the present invention may be administered orally, parenterally, by inhalation spray, rectally, or topically in dosage unit formulations containing conventional nontoxic pharmaceutically acceptable carriers, adjuvants, and vehicles as desired. The term parenteral as used herein includes subcutaneous injections, intravenous, intramuscular, intrasternal injection, or infusion techniques.

Injectable preparations, for example, sterile injectable aqueous or oleaginous suspensions may be formulated according to the known art using suitable dispersing or wetting agents and suspending agents. The sterile injectable preparation may also be a sterile injectable solution or suspension in a nontoxic parenterally acceptable diluent or solvent, for example, as a solution in 1,3-butanediol. Among the acceptable vehicles and solvents that may be employed are water, Ringer's solution, and isotonic sodium chloride solution. In addition, sterile, fixed oils are conventionally employed as a solvent or suspending

medium. For this purpose any bland fixed oil may be employed including synthetic mono- or diglycerides. In addition, fatty acids such as oleic acid find use in the preparation of injectables.

Suppositories for rectal administration of the drug can be prepared by mixing the drug with a suitable nonirritating excipient such as cocoa butter and polyethylene glycols which are solid at ordinary temperatures but liquid at the rectal temperature and will therefore melt in the rectum and release the drug.

Solid dosage forms for oral administration may include capsules, tablets, pills, powders, and granules. In such solid dosage forms, the active compound may be admixed with at least one inert diluent such as sucrose lactose or starch. Such dosage forms may also comprise, as is normal practice, additional substances other than inert diluents, e.g., lubricating agents, such as magnesium stearate. In the case of capsules, tablets, and pills, the dosage forms may also comprise buffering agents. Tablets and pills can additionally be prepared with enteric coatings.

Liquid dosage forms for oral administration may include pharmaceutically acceptable emulsions, solutions, suspensions, syrups, and elixirs containing inert diluents commonly used in the art, such as water. Such compositions may also comprise adjuvants, such as wetting agents, emulsifying and suspending agents, and sweetening, flavoring, and perfuming agents.

The novel polypeptides and salts thereof of the invention can be utilized effectively as vasorelaxant agents. Accordingly, the present invention further relates to vasorelaxant compositions comprising a novel

polypeptide having the general formula I or salts thereof as an active component.

The polypeptides of the invention may be prepared by a synthetic method of elongation of a peptide chain through condensation of one amino acid by one, or by a method of coupling fragments consisting of two or several amino acids, or by a combination of these methods in accordance with conventional peptide synthesis methods.

The condensation of two amino acids, the condensation of an amino acid with a peptide or the condensation of one peptide with another peptide may be effected in accordance with conventional condensation methods such as azide method, mixed acid anhydride method, DCC (dicyclohexylcarbodiimide) method, active ester method (p-nitrophenyl ester method, N-hydroxysuccinic acid imide ester method, cyanomethyl ester method and the like), Woodward reagent K method, DCC-HOBT(1-hydroxy-benzotriazole) method and the like. These condensation reactions may be done by either solution methods or solid phase synthetic methods. When the peptide chain is elongated by solid phase method, the C-terminal amino acid was linked to an insoluble carrier. As the insoluble carrier, any of which can produce a detachable bond by reacting with a carboxyl group in a C-terminal amino acid may be used, and the examples thereof involve, for example, halomethyl resins such as chloromethyl resin, bromomethyl resin and the like, hydroxy-methyl resin, aminomethyl resin, benzhydrylamine resin, t-alkyloxycarbonyl hydrazide resin.

As conventional polypeptide synthesis, branched chain amino and carboxyl groups at alpha and omega positions in amino acids may be protected/deprotected if necessary. The protecting groups for amino groups which can be used involve, for example, benzyloxycarbonyl (Z), o-chlorobenzyloxycarbonyl ((2-Cl)Z), p-nitrobenzyl-oxycarbonyl ($Z(NO_2)$), p-methoxybenzyloxycarbonyl (Z(OMe)), t-butoxycarbonyl (Boc), t-amyloxycarbonyl (Aoc), isobornyloxycarbonyl, admantyloxycarbonyl, 2-(4-biphenyl)-2- propyloxycarbonyl (Bpoc), 9-fluorenyl-methoxycarbonyl (Fmoc), methylsulfonylethoxycarbonyl (Msc), trifluoroacetyl, phthalyl, formyl, 2-nitrophenylsulfenyl (NPs), diphenylphosphinothioyl (Ppt), and dimethylphosphinothioyl (Mpt).

The examples of the protecting groups for carboxyl groups involve, for example, benzyl ester (OBzl), cyclohexyl ester, 4-nitrobenzyl ester ($OBzl(NO_2)$), t-butyl ester (OtBu), 4-pyridylmethyl ester (OPic) and the like.

In the course of the synthesis of the present peptides, specific amino acids having functional groups other than amino and carboxyl groups in the branched chain such as arginine, cysteine, serine, and the like may be protected, if necessary, with suitable protecting group. It is preferable that for example, the guanidino group ($N^G$) in arginine may be protected with nitro,p-toluene-sulfonyl (Tos), benzyloxycarbonyl (Z), adamantyloxycarbonyl (Adoc), p-methoxybenzenesulfonyl, 4-methoxy-2,6-dimethylbenzenesulfonyl (Mds), 1,3,5-trimethylphenyl-sulfonyl (Mts) and the like, and the thiol group in

cysteine may be protected with benzyl, p-methoxybenzyl, triphenylmethyl, acetamidomethyl, ethylcarbamyl, 4-methylbenzyl (4-MeBzl) 2,4,6-trimethylbenzyl (Tmb) and the like, and the hydroxyl group in serine may be protected with benzyl (Bzl), t-butyl, acetyl, tetrahydropyranyl and the like.

The compounds of the invention are prepared by standard solid phase peptide synthesis conditions as described in "Solid Phase Peptide Synthesis" by John M. Stewart and Janis D. Young, Second Edition (1984) and illustrated in Examples 1 and 2 in the experimental section.

The compounds of the invention may also be prepared by partial solid phase synthesis, frequent condensation methods and classical solution methods as exemplified by the methods described in "Peptide Synthesis", Second Edition, M. Bodanszky, Y. S. Klausner, and M. A. Ondetti (1976).

The foregoing may be better understood by reference to the following examples which are provided for illustration and not limitation of the practice of the invention.

## Example 1

### Seryl-Seryl-Cysteinyl-Phenylalanyl-Glycyl-Glycyl-Arginyl-Isoleucyl-Aspartyl-Arginyl-Isoleucyl-Cysteinyl-Phenylalanyl-Arginyl-protected peptide resin

0.5 g - 1.0 g N-alpha-t-Boc Arginyl ($N^G$Tos)-O Merrifield resin is placed in a solid phase peptide synthesis vessel and amino acids are attached to the peptide resin sequentially in the following order: Boc-Phe, Boc-Cys(4-MeBzl), Boc-Ile, Boc-Arg($N^G$Tos),

Boc-Asp (ß-cyclohexyl), Boc-Ile, Boc-Arg ($N^G$Tos), Boc-Gly, Boc-Gly, Boc-Phe, Boc-Cys(4-MeBzl), Boc-Ser(Bzl), Boc-Ser(Bzl) according to the protocol in Agenda A to yield, the protected peptide resin Ser(Bzl)-Ser(Bzl)-Cys(4-MeBzl)-Phe-Gly-Gly-Arg($N^G$Tos)-Ile Asp(ß-cyclohexyl)-Arg ($N^G$Tos)-Ile-Cys(4-MeBzl)-Phe-Arg ($N^G$Tos)-O Resin. Following the synthesis, the protected peptide resin was removed from the reaction vessel by washing the resin three times with 20 ml DMF into a 60 ml. sintered glass funnel, followed by washing the resin 3 times with 30 ml $CH_2Cl_2$. The resin is dried in vacuo 3 hours, then weighed.

<u>Agenda A</u>

1. Deblock: 50% Trifluoroacetic acid (TFA) in $CH_2Cl_2$ containing 2% Anisole, 0.5% Ethanedithiol (v/v/v).

2. Neutralization: 10% Diisopropylethylamine (DIEA) in $CH_2Cl_2$ (v/v).

3. Single Coupling: 0.2 M Boc-amino acid derivative in N,N-dimethylformamide (DMF), 0.2 M diisopropylcarbodiimide in $CH_2Cl_2$, reaction time 45 minutes.

4. Resin Clean Up: 10% DIEA in $CH_2Cl_2$.

5. Single Coupling: 0.2 M Boc-amino acid derivative (same as Step 3) in DMF; 0.2 M diisopropylcarbodiimide in $CH_2Cl_2$ reaction time: 45 minutes.

6. Cap: 0.3 M Acetylimidazole in DMF.

7. Go to next amino acid residue.

8. Upon attachment of the final amino acid to the growing peptide chain, the protecting group (t-Boc) is removed as in Step 1.

## Example 2

### Preparation of Seryl-Seryl-Cysteinyl-Phenylalanyl-Glycyl-Glycyl-Arginyl-Isoleucyl-Aspartyl-Arginyl-Isoleucyl Cysteinyl-Phenylalanyl-Arginyl Cyclic Disulfide

0.5 g protected peptide resin of Example 1 was treated with 2 ml anisole and 15 ml liquid hydrogen fluoride (HF) for 60 minutes at 0°C. The HF and anisole was removed from the resin *in vacuo* and the resin was washed with 2 x 25 ml portions of ethyl ether or ethyl acetate under a nitrogen atmosphere. The crude peptide was extracted from the resin by treatment with 6 x 50 ml portions of 20% aqueous acetic acid. The acetic acid solution was diluted to 1,500 ml with distilled water and the pH of the solution was adjusted to 8.3 with 80 ml concentrated ammonium hydroxide. The solution was oxidized with an excess of 0.01N $K_3Fe(CN)_6$. The resultant yellow solution was stirred for 60 minutes at room temperature while the pH was maintained at 8.3.

The excess ferri- and ferro-cyanide ions were removed from the solution by first adjusting the pH to 5.5 with 50 ml glacial acetic acid and then adding 10 ml of the anion exchange resin BioRad AG 3X-4A ($Cl^-$ cycle). The mixture was stirred for 20 minutes at room temperature. The resin was filtered off and washed with 3 x 25 ml portions of 20% aqueous acetic acid. The combined aqueous solution was applied to a column containing 300 g XAD-16 molecular adsorbent resin. The sample was desalted by first washing the column with 3 l distilled water. The sample was eluted from the column with 1,500 ml 50% aqueous ethanol. The combined ethanol fractions were concentrated to approximately 100 ml _in vacuo_, taken up in an additional 100 ml distilled water and lyophilized to a dry amorphous powder. The dry powder is purified by High Performance Liquid Chromatography (HPLC) using 0.1% aqueous trifluoroacetic acid and acetonitrile as organic modifier. The preparative analyses were performed using a Dynamax (RAININ) $C_{18}$ Reverse-phase Column 2.1 cm i.d. x 25 cm length and flow rate of 12-15 ml/min. The sample was purified by gradient elution. Analytical HPLC analysis was performed using a VYDAC 218 TP 10u, $C_{18}$ Reverse-phase Column 0.46 cm i.d. x 20 cm length. Here was obtained 20 mg (6%) of the title compound. Amino Acid Analysis: Asp(1.03), Ser(1.60), Gly(2.00), Cys (0.88), Ile(1.94), Phe(2.00), Arg(3.26); $FAB^+$ $(M+H)^+$ at M/Z: 1615.

## Example 3

### Arginyl-Arginyl-Seryl-Seryl-Cysteinyl-Phenylalanyl-Glycyl Glycyl-Arginyl-Isoleucyl-Aspartyl-Arginyl-Isoleucyl-Cysteinyl-Phenylalanyl-Arginyl-Cyclic Disulfide

0.39 g protected peptide resin, prepared from N-alpha-t-Boc-L-Arginine ($N^G$Tos) Merrifield resin and the amino acids attached sequentially in the following order: Boc-Phe, Boc-Cys(4-MeBzl), Boc-Ile, Boc-Arg($N^G$Tos), Boc-Asp (ß-cyclohexyl), Boc-Ile, Boc-Arg ($N^G$Tos), Boc-Gly, Boc-Gly, Boc-Phe, Boc-Cys (4-MeBzl), Boc-Ser (Bzl), Boc-Ser(Bzl), Boc-Arg ($N^G$Tos), Boc-Arg($N^G$Tos), was cleaved from the resin and purified as in Example 2. The title compound was obtained as a white powder. 31 mg.
$FAB^+$ $(M+H)^+$ at M/Z: 1927.
Amino acid analysis: Arg(5.05), Asp(1.00), Cys(2.21), Gly (2.11), Ile(1.77), Phe(1.82), Ser(1.73).

## Example 4

### Arginyl-Arginyl-Seryl-Seryl-Cysteinyl-Phenylalanyl-Glycyl-Glycyl Arginyl-Isoleucyl-Aspartyl-Arginyl-Isoleucyl-Cysteinyl Cyclic Disulfide

0.58 g protected peptide resin, prepared from N-alpha-t-Boc-Cys(4-MeBzl) Merrifield resin and the amino acids attached sequentially in the following order: Boc-Ile, Boc-Arg ($N^G$Tos), Boc-Asp(ß-cyclohexyl), Boc-Ile, Boc-Arg ($N^G$Tos), Boc-Gly, Boc-Gly, Boc-Phe, Boc-Cys(4-MeBzl), Boc-Ser(Bzl), Boc-Ser(Bzl), Boc-Arg ($N^G$Tos), Boc-Arg ($N^G$Tos), was cleaved from the resin and purified as in

Example 2. The title compound was obtained as a white powder. 16 mg.

$FAB^+$ $(M+H)^+$ at M/Z: 1624.

Amino acid analysis: Arg(3.9), Asp(1.0), Cys(1.3), Gly(2.2), Ile (1.9), Phe(1.2), Ser(1.7).

## Example 5

### Seryl-Cysteinyl-Phenylalanyl-Glycyl-Glycyl-Arginyl-Isoleucyl-Aspartyl-Arginyl-Isoleucyl-Cysteinyl-Phenylalanyl-Arginyl Cyclic Disulfide

0.45 g protected peptide resin, prepared from N-alpha-t-Boc-Arg ($N^G$Tos) Merrifield resin and the amino acids added sequentially in the following order: Boc-Phe, Boc-Cys(4-MeBzl), Boc-Ile, Boc-Arg ($N^G$Tos), Boc-Asp-(ß-cyclohexyl), Boc-Ile, Boc-Arg ($N^G$Tos)), Boc-Gly, Boc-Gly, Boc-Phe, Boc-Cys(4-MeBzl), Boc-Ser(Bzl), was treated with HF(liquid) and purified as described in Example 2. The title compound was obtained as a white powder. 23 mg.

$FAB^+$ $(M+H)^+$ at M/Z: 1529.

Amino acid analysis: Arg(3.0), Asp(1.0), Cys(1.3), Gly(2.2), Ile(1.8), Phe(2.0), Ser(0.8).

## Example 6

### Acetyl-Cysteinyl-Phenylalanyl-Glycyl-Glycyl-Arginyl-Isoleucyl-Aspartyl-Arginyl-Isoleucyl-Cysteinyl-Phenylalanyl-Arginyl Cyclic Disulfide

0.7 g protected peptide resin, prepared from N-alpha-t-Boc-Arg ($N^G$Tos) Merrifield resin and the amino acids added sequentially in the following order:

Boc-Phe, Boc-Cys(4-MeBzl), Boc-Ile, Boc-Arg ($N^G$Tos), Boc-Asp (ß-cyclohexyl), Boc-Ile, Boc-Arg ($N^G$ Tos), Boc-Gly, Boc-Gly, Boc-Phe, Boc-Cys(4-MeBzl). Acetylation was accomplished with acetic anhydride in pyridine. The protected peptide was treated with HF (liquid) and purified as described in Example 2. The title compound was obtained as a white powder.
$FAB^+$ $(M+H)^+$ at M/Z: 1483.
Amino Acid analysis: Arg(1.85), Asp(0.94), Cys(0.8), Gly (2.02), Ile(1.48), Phe(2.00).

Example 7

Beta-Mercaptopropionyl-Phenylalanyl-Glycyl-Glycyl-Arginyl--Isoleucyl-Aspartyl-Arginyl-Isoleucyl-Cysteinyl-Phenylalanyl-Arginyl Cyclic Disulfide

0.475 g protected peptide resin, prepared from N-alpha-t-Boc-Arg ($N^G$Tos) Merrifield resin and the amino acids added sequentially in the following order: Boc-Phe, Boc-Cys(4-MeBzl), Boc-Ile, Boc-Arg ($N^G$Tos), Boc-Asp(ß-cyclohexyl), Boc-Ile, Boc-Arg ($N^G$Tos), Boc-Gly, Boc-Gly, Boc-Phe, Mercaptoprionic acid (Mpa), was cleaved from the resin and purified as described in Example 2. The title compound was obtained as a white powder. 33 mg.
$FAB^+$ $(M+H)^+$ at M/Z: 1425.
Amino acid analysis: Arg(2.9), Asp(1.0), Cys(0.7), Gly(2.2), Ile(1.8), Phe(2.0).

## Example 8

Cysteinyl-Phenylalanyl-Glycyl-Glycyl-Arginyl-Isoleucyl-Aspartyl-Arginyl-Isoleucyl-Cysteinyl Cyclic Disulfide

0.500 g protected peptide resin, prepared from N-alpha-t-Boc-Cys(4-MeBzl) Merrifield resin and the amino acids were added sequentially in the following order: Boc-Ile, Boc-Arg ($N^G$Tos), Boc-Asp (ß-cyclohexyl), Boc-Ile, Boc-Arg ($N^G$Tos), Boc-Gly, Boc-Gly, Boc-Phe, Boc-Cys(4-MeBzl) was cleaved from the resin and purified as described in Example 2. The title compound was obtained as a white powder. 17 mg.
$FAB^+$ $(M+H)^+$ at M/Z: 1137.
Amino acid analysis: Arg(1.8), Asp(0.9), Cys(1.1), Gly(2.2), Ile(1.4), Phe(0.9).

## Example 9

Cysteinyl-Phenylalanyl-Glycyl-Arginyl-Isoleucyl-Aspartyl-Arginyl-Isoleucyl-Cysteinyl Cyclic Disulfide

0.400 g protected peptide resin, prepared from N-alpha-t-Boc Cys(4-MeBzl) Merrifield resin and the amino acids added sequentially in the following order: Boc-Ile, Boc-Arg ($N^G$Tos), Boc-Asp (ß-cyclohexyl), Boc-Ile, Boc-Arg ($N^G$Tos) Boc-Gly, Boc-Phe, Boc-Cys(4-MeBzl), was cleaved with HF (liquid) and purified as described in Example 2. The title compound was obtained as a white powder. 13 mg.
$FAB^+$ $(M+H)^+$ at M/Z: 1080.
Amino acid analysis: Arg(1.9), Asp(1.0), Cys(1.4), Gly(1.0), Ile(1.8), Phe(1.0).

Example 10

Cysteinyl-Phenylalanyl-D-Alanyl-Glycyl-Arginyl-Isoleucyl-Aspartyl-Arginyl-Isoleucyl-Cysteinyl Cyclic Disulfide

0.400 g protected peptide resin, prepared from N-alpha-t-Boc-Cys(4-MeBzl) Merrifield resin and the amino acids added sequentially in the following order: Boc-Ile, Boc-Arg ($N^G$Tos), Boc-Asp(ß-cyclohexyl), Boc-Ile, Boc-Arg ($N^G$Tos), Boc-Gly, Boc-D-Ala, Boc-Phe, Boc-Cys(4-MeBzl), was cleaved with HF (liquid) and purified as described in Example 2. The title compound was obtained as a white powder. 6 mg.
$FAB^+$ $(M+H)^+$ at M/Z: 1151.
Amino acid analysis: Ala(0.6), Arg(2.2), Asp(1.0), Cys(1.6), Gly(1.3), Ile(1.9), Phe(0.7).

Example 11

Seryl-Seryl-Cysteinyl-Phenylalanyl-Glycyl-Prolyl-Arginyl-Isoleucyl-Aspartyl-Arginyl-Isoleucyl-Cysteinyl Cyclic Disulfide

0.400 g protected peptide resin, prepared from N-alpha-t-Boc-Cys(4-MeBzl) Merrifield resin and the amino acids added sequentially in the following order: Boc-Ile, Boc-Arg ($N^G$Tos), Boc-Asp(ß-cyclohexyl), Boc-Ile, Boc-Arg ($N^G$Tos), Boc-Pro, Boc-Gly, Boc-Phe, Boc-Cys(4-MeBzl), Boc-Ser(Bzl), Boc-Ser(Bzl), was treated with HF (liquid) and purified as described in Example 2. The title compound was obtained as a white powder. 18 mg.
$FAB^+$ $(M+H)^+$ at M/Z: 1351.
Amino acid analysis: Arg(2.1), Asp(1.0), Cys(1.8), Gly(1.1), Ile(1.8), Phe(1.0), Ser(1.4), Pro(1.0).

Example 12

Seryl-Seryl-Cysteinyl-Phenylalanyl-Glycyl-Glycyl-Arginyl-Isoleucyl-Aspartyl-Arginyl-Isoleucyl-Cysteinyl Cyclic Disulfide

0.300 g protected peptide resin, prepared from N-alpha-t-Boc-Cys(4-MeBzl) Merrifield resin and the amino acids added sequentially in the following order: Boc-Ile, Boc-Arg ($N^G$Tos), Boc-Asp (ß-cyclohexyl), Boc-Ile, Boc-Arg ($N^G$Tos), Boc-Gly, Boc-Gly, Boc-Phe, Boc-Cys(4-MeBzl), Boc-Ser(Bzl), Boc-Ser(Bzl) was treated with liquid HF and purified as described in Example 2. The title compound was obtained as a white powder. 31 mg.
$FAB^+$ $(M+H)^+$ at M/Z: 1311.
Amino acid analysis: Arg(2.0), Asp(0.8), Cys(1.8), Gly(1.7), Ile(1.8), Phe(0.9), Ser(1.4).

Example 13

Seryl-Seryl-Cysteinyl-Phenylalanyl-Glycyl-Glycyl-Arginyl-Isoleucyl-Glycyl-Arginyl-Isoleucyl-Cysteinyl-Phenylalanyl-Arginyl Cyclic Disulfide

0.75 g protected peptide resin, prepared from N-alpha-t-Boc-Arg ($N^G$Tos) Merrifield resin and the amino acids added sequentially in the following order: Boc-Phe, Boc-Cys(4-MeBzl), Boc-Ile, Boc-Arg ($N^G$Tos), Boc-Gly, Boc-Ile, Boc-Arg ($N^G$Tos), Boc-Gly, Boc-Gly, Boc-Phe, Boc-Cys-(4-MeBzl), Boc-Ser(Bzl), Boc-Ser(Bzl), was treated with liquid HF and purified as described in Example 2. The title compound was obtained as a white powder. 55 mg.
$FAB^+$ $(M+H)^+$ at M/Z: 1556.
Amino acid analysis: Arg(3.06), Cys(2.01), Gly(3.33), Ile(2.13), Phe(2.13), Ser(1.69).

## Example 14

Seryl-Seryl-Cysteinyl-Phenylalanyl-Glycyl-Glycyl-Lysyl-Isoleucyl-Aspartyl-Arginyl-Isoleucyl-Cysteinyl-Phenylalanyl-Arginyl Cyclic Disulfide

0.73 g protected peptide resin, prepared from N-alpha-t-Boc-Arg ($N^G$Tos) Merrifield resin and the amino acids added sequentially in the followng order: Boc-Phe, Boc-Cys(4-MeBzl), Boc-Ile, Boc-Arg ($N^G$Tos), Boc-Asp(ß-cyclohexyl), Boc-Ile, Boc-Lys (2Cl-Z), Boc-Gly, Boc-Gly, Boc-Phe, Boc-Cys(4-MeBzl), Boc-Ser(Bzl), Boc-Ser(Bzl), was treated with HF (liquid) and purified as described in Example 2. The title compound was obtained as a white powder. 74 mg.
$FAB^+$ $(M+H)^+$ at M/Z: 1586.
Amino acid analysis: Arg(1.94), Asp(1.06), Cys(1.85), Gly(2.18), Ile(2.00), Lys(0.92), Phe(1.96), Ser(1.76).

## Example 15

Seryl-Seryl-Cysteinyl-Phenylalanyl-Glycyl-Glycyl-Arginyl-Isoleucyl-Aspartyl-Lysyl-Isoleucyl-Cysteinyl-Phenylalanyl-Arginyl Cyclic Disulfide

0.96 g protected peptide resin, prepared from N-alpha-t-Boc-Arg-($N^G$Tos) Merrifield resin and the amino acids added sequentially in the following order: Boc-Phe, Boc-Cys(4-MeBzl), Boc-Ile, Boc-Lys ((2-Cl)Z), Boc-Asp(ß-cyclohexyl), Boc-Ile, Boc-Arg ($N^G$Tos), Boc-Gly, Boc-Gly, Boc-Phe, Boc-Cys(4-MeBzl), Boc-Ser(Bzl), Boc-Ser(Bzl), was treated with HF (liquid) and purified as described in Example 2. The title compound was obtained as a white powder. 50 mg.
$FAB^+$ $(M+H)^+$ at M/Z: 1586.
Amino acid analysis: Arg(2.02), Asp(1.10), Cys(1.87), Gly(2.16), Ile(2.01), Lys(0.98), Phe(2.05), Ser(1.83).

## Example 16

**Seryl-Seryl-Cysteinyl-Phenylalanyl-Alanyl-Glycyl-Arginyl-Isoleucyl-Aspartyl-Arginyl-Isoleucyl-Cysteinyl-Phenylalanyl-Arginyl Cyclic Disulfide**

0.81 g protected peptide resin, prepared from N-alpha-t-Boc-Arg($N^G$Tos) Merrifield resin and the amino acids added sequentially in the following order: Boc-Phe, Boc-Cys(4-MeBzl), Boc-Ile, Boc-Arg ($N^G$Tos), Boc-Asp(ß-cyclohexyl), Boc-Ile, Boc-Arg ($N^G$Tos), Boc-Gly, Boc-Ala, Boc-Phe, Boc-Cys(4-MeBzl), Boc-Ser(Bzl), Boc-Ser(Bzl), was treated with HF (liquid) and purified as described in Example 2. The title compound was obtained as a white powder. 51 mg.
$FAB^+$ $(M+H)^+$ at M/Z: 1628.
Amino acid analysis: Ala(1.04), Arg(3.01), Asp(1.01), Cys(1.78), Gly(1.17), Ile(1.94), Phe(2.01), Ser(1.76).

## Example 17

**Seryl-Seryl-Cysteinyl-Phenylalanyl-(D)-Alanyl-Glycyl-Arginyl-Isoleucyl-Aspartyl-Arginyl-Isoleucyl-Cysteinyl-Phenylalanyl-Arginyl Cyclic Disulfide**

1.3 g protected peptide resin, prepared from N-alpha-t-Boc-Arg ($N^G$Tos) Merrifield resin and the amino acids added sequentially in the following order: Boc-Phe, Boc-Cys(4-MeBzl), Boc-Ile, Boc-Arg ($N^G$Tos), Boc-Asp(ß-cyclohexyl), Boc-Ile, Boc-Arg ($N^G$Tos), Boc-Gly, Boc-D-Ala, Boc-Phe, Boc-Cys(4-MeBzl), Boc-Ser(Bzl), Boc-Ser(Bzl), was treated with HF (liquid) and purified as described in Example 2. The title compound was obtained as a white powder. 69 mg.
$FAB^+$ $(M+H)^+$ at M/Z: 1628.
Amino acid analysis: Ala(1.05), Arg(3.01), Asp(1.06), Cys(1.91), Gly(1.10), Ile(2.00), Phe(2.06), Ser(1.78).

## Example 18

Seryl-Seryl-Cysteinyl-Phenylalanyl-Glycyl-Glycyl-Arginyl-Glycyl-Aspartyl-Arginyl-Isoleucyl-Cysteinyl-Phenylalanyl-Arginyl Cyclic Disulfide

1.1 g protected peptide resin, prepared from N-alpha-t-Boc-Arg ($N^G$Tos) Merrifield resin and the amino acids added sequentially in the following order: Boc-Phe, Boc-Cys (4-MeBzl), Boc-Ile, Boc-Arg ($N^G$Tos), Boc-Asp(ß-cyclohexyl), Boc-Gly, Boc-Arg ($N^G$Tos), Boc-Gly, Boc-Gly, Boc-Phe, Boc-Cys(4-MeBzl), Boc-Ser(Bzl), Boc-Ser(Bzl), was treated with HF (liquid) and purified as described in Example 2. The title compound was obtained as a white powder. 60 mg.
$FAB^+$ $(M+H)^+$ at M/Z: 1559.
Amino acid analysis: Arg(3.09), Asp(1.07), Cys(1.80), Gly(3.22), Ile(1.03), Phe(2.11), Ser(1.94).

## Example 19

Cysteinyl-Phenylalanyl-Glycyl-Prolyl-Arginyl-Isoleucyl-Aspartyl-Arginyl-Isoleucyl-Cysteinyl Cyclic Disulfide

0.5 g protected peptide resin, prepared from N-alpha-t-Boc-Cys(4-MeBzl) Merrifield resin and the amino acids added sequentially in the following order: Boc-Ile, Boc-Arg, ($N^G$Tos), Boc-Asp(ß-cyclohexyl), Boc-Ile, Boc-Arg ($N^G$Tos), Boc-Pro, Boc-Gly, Boc-Phe, Boc-Cys (4-MeBzl), was treated with HF (liquid) and purified as described in Example 2.. The title compound was obtained as a white powder. 11 mg.
$FAB^+$ $(M+H)^+$ at M/Z: 1177.
Amino acid analysis: Arg(2.14), Asp(1.05), Cys(1.95), Gly(1.16), Ile(1.89), Pro(0.88), Phe(0.95).

## Example 20

**Seryl-Seryl-Cysteinyl-Phenylalanyl-Glycyl-Prolyl-Arginyl-Isoleucyl-Aspartyl-Arginyl-Isoleucyl-Cysteinyl-Phenylalanyl-Arginyl Cyclic Disulfide**

0.74 g protected peptide resin, prepared from N-alpha-t-Boc-Arg ($N^G$Tos) Merrifield resin and the amino acids added sequentially in the following order: Boc-Phe, Boc-Cys(4-MeBzl), Boc-Ile, Boc-Arg ($N^G$Tos), Boc-Asp(ß-cyclohexyl), Boc-Ile, Boc-Arg ($N^G$Tos), Boc-Pro, Boc-Gly, Boc-Phe, Boc-Cys(4-MeBzl), Boc-Ser(Bzl), Boc-Ser(Bzl), was treated with HF (liquid) and purified as described in Example 2. The title compound was obtained as a white powder. 6 mg.
$FAB^+$ $(M+H)^+$ at M/Z: 1655.
Amino acid analysis: Arg(2.82), Asp(1.01), Cys(1.54), Gly(1.42), Ile(2.00), Pro(1.09), Phe(2.04), Ser(1.34).

## Example 21

**Seryl-Cysteinyl-Phenylalanyl-Glycyl-Glycyl-Arginyl-Isoleucyl-Aspartyl-Arginyl-Isoleucyl-Cysteinyl-Cyclic Disulfide**

0.5 g protected peptide resin, prepared from N-alpha-t-Boc-Cys(4-MeBzl) Merrifield resin and the amino acids added sequentially in the following order: Boc-Ile, Boc-Arg ($N^G$Tos), Boc-Asp(ß-cyclohexyl), Boc-Ile, Boc-Arg ($N^G$Tos), Boc-Gly, Boc-Gly, Boc-Phe, Boc-Cys (4-MeBzl), Boc-Ser(Bzl), was treated with HF (liquid) and purified as described in Example 2. The title compound was obtained as a white powder. 22 mg.
$FAB^+$ $(M+H)^+$ at M/Z: 1224.
Amino acid analysis: Arg(1.97), Asp(1.12), Cys(2.01), Gly(2.25), Ile(1.82), Phe(1.06), Ser(0.97).

## Example 22

Cysteinyl-Phenylalanyl-Glycyl-Glycyl-Arginyl-Isoleucyl-Aspartyl-Arginyl-Isoleucyl-Cysteinyl-Phenylalanyl-Arginyl Cyclic Disulfide

0.49 g protected peptide resin, prepared from N-alpha-t-Boc-Arg ($N^G$Tos) Merrifield resin and the amino acids added sequentially in the following order: Boc-Phe, Boc-Cys(4-MeBzl), Boc-Ile, Boc-Arg ($N^G$Tos), Boc-Asp(ß-cyclohexyl), Boc-Ile, Boc-Arg ($N^G$Tos), Boc-Gly, Boc-Gly, Boc-Phe, Boc-Cys(4-MeBzl), was treated in HF (liquid) and purified as described in Example 2. The title compound was obtained as a white powder. 59mg. $FAB^+$ ($M+H^+$) at M/Z: 1441.

Amino acid analysis: Arg(3.09), Asp(1.00), Cys(1.7), Gly(2.0), Ile(1.98), Phe(1.85).

## Example 23

Cysteinyl-Phenylalanyl-Glycyl-Glycyl-Arginyl-Isoleucyl-Aspartyl-Arginyl-Isoleucyl-Cysteinyl-Asparaginyl-Seryl-Phenylalanyl-Arginyl-Cyclic Disulfide

0.5 g protected peptide resin prepared from N-alpha-t-Boc-Arg ($N^G$Tos) Merrifield resin and the amino acids added sequentially in the following order: Boc-Phe, Boc-Ser(Bzl), Boc-Asn, Boc-Cys(4-MeBzl), Boc-Ile, Boc-Arg ($N^G$Tos), Boc-Asp(ß-cyclohexyl), Boc-Ile, Boc-Arg ($N^G$Tos), Boc-Gly, Boc-Gly, Boc-Phe, Boc-Cys(4-MeBzl), was treated with HF (liquid) and purified as described in Example 2. The title compound was obtained as a white powder. 24 mg.

$FAB^+$ ($M+H^+$) at M/Z: 1641.

Amino acid analysis: Arg(2.95), Asx(2.00), Cys(1.9), Gly(2.00), Ile(1.8), Phe(1.9), Ser(0.8).

## Example 24

Seryl-Seryl-Cysteinyl-Phenylalanyl-Glycyl-Glycyl-Arginyl-Isoleucyl-Aspartyl-Arginyl-Isoleucyl-Cysteinyl-Asparaginyl-Seryl-Phenylalanyl-Arginyl-Cyclic Disulfide

0.3 g protected peptide resin, prepared from N-alpha-t-Boc-Arg ($N^G$Tos) Merrifield resin and the amino acids added sequentially in the following order: Boc-Phe, Boc-Ser(Bzl), Boc-Asn, Boc-Cys(4-MeBzl), Boc-Ile, Boc-Arg ($N^G$Tos), Boc-Asp(ß-cyclohexyl), Boc-Ile, Boc-Arg ($N^G$Tos), Boc-Gly, Boc-Gly, Boc-Phe, Boc-Cys(4-MeBzl), Boc-Ser(Bzl), Boc-Ser(Bzl), was treated with HF (liquid) and purified as described in Example 2. The title compound was obtained as a white powder. 10 mg.
$FAB^+$ ($M+H^+$) at M/Z: 1817.
Amino acid analysis: Asx(2.00), Arg(3.10), Ser(2.5), Gly(2.00), Cys(1.6), Ile(1.85), Phe(1.75).

## Example 25

Seryl-Seryl-Penicillaminyl-Phenylalanyl-Glycyl-Glycyl-Arginyl-Isoleucyl-Aspartyl-Arginyl-Isoleucyl-Cysteinyl-Phenylalanyl-Arginyl-Cyclic Disulfide

0.5 g protected peptide resin, prepared from N-alpha-t-Boc-Arg ($N^G$Tos) Merrifield resin and the amino acids added sequentially the following order: Boc-Phe, Boc-Cys(4-MeBzl), Boc-Ile, Boc-Arg ($N^G$Tos), Boc-Asp (ß-cyclohexyl), Boc-Ile, Boc-Arg ($N^G$Tos), Boc-Gly, Boc-Gly, Boc-Phe, Boc-Pen(4-MeBzl), Boc-Ser(Bzl), Boc-Ser(Bzl), was treated with HF (liquid) and purfiied as described in Example 2. The title compound was obtained as a white powder. 26 mg.
$FAB^+$ ($M+H^+$) at M/Z: 1643.
Amino acid analysis: Asp(1.00), Ser(1.7), Gly(2.00), Cys(0.9), Pen(0.5), Ile(1.95), Phe(0.85), Arg(2.95).

## Example 26

**Seryl-Seryl-Cysteinyl-Phenylalanyl-Glycyl-Glycyl-Arginyl-Isoleucyl-Aspartyl-Arginyl-Isoleucyl-Penicillaminyl-Phenylalanyl-Arginyl-Cyclic Disulfide**

0.4 g protected peptide resin, prepared from N-alpha-t-Boc-Arg ($N^G$Tos) Merrifield resin and the amino acids added sequentially in the following order: Boc-Phe, Boc-Pen(4-MeBzl), Boc-Ile, Boc-Arg($N^G$Tos), Boc-Asp(ß-cyclohexyl), Boc-Ile, Boc-Arg ($N^G$Tos), Boc-Gly, Boc-Gly, Boc-Phe, Boc-Cys(4-MeBzl), Boc-Ser(Bzl), Boc-Ser(Bzl), was treated with HF (liquid) and purified as described in Example 2.

## Example 27

**Seryl-Seryl-Cysteinyl-ß-Napthylalanyl-Glycyl-Glycyl-Arginyl-Isoleucyl-Aspartyl-Arginyl-Isoleucyl-Cysteinyl-Phenylalanyl-Arginyl-Cyclic-Disulfide**

0.5 g protected peptide resin, prepared from N-alpha-t-Boc-Arg ($N^G$Tos) Merrifield resin and the amino acids added sequentially in the followin order: Boc-Phe, Boc-Cys(4-MeBzl), Boc-Ile, Boc-Arg ($N^G$Tos), Boc-Asp-(ß-cyclohexyl), Boc-Ile, Boc-Arg($N^G$Tos), Boc-Gly, Boc-Gly, Boc-ß-Nal, Boc-Cys(4-MeBzl), Boc-Ser(Bzl), Boc-Ser(Bzl), was treated with HF (liquid) and purified as described in Example 2 yielding a white powder. 10 mg.

$FAB^+$ ($M+H^+$) at M/Z: 1664

Amino acid analysis: Asp(0.9), Ser(1.6), Gly(1.9), Cys(1.3), Ile(1.7), Phe(1.0), Arg(2.9).

## Example 28

Seryl-Seryl-Cysteinyl-Phenylalanyl-Glycyl-Glycyl-Glycyl-Isoleucyl-Aspartyl-Arginyl-Isoleucyl-Cysteinyl-Phenylalanyl-Arginyl-Cyclic-Disulfide

0.5 g protected peptide resin, prepared from N-alpha-t-Boc-Arg ($N^G$Tos) Merrifield resin and the amino acids added sequentially in the following order: Boc-Phe, Boc-Cys-(4-MeBzl), Boc-Ile, Boc-Arg ($N^G$Tos), Boc-Asp(ß-cyclohexyl), Boc-Ile, Boc-Gly, Boc-Gly, Boc-Gly, Boc-Phe, Boc-Cys-(4-MeBzl), Boc-Ser(Bzl), Boc-Ser(Bzl), was treated with HF (liquid) and purified as described in Example 2 yielding a white powder. 17 mg.

$FAB^+$ ($M+H^+$) at M/Z: 1516.

Amino acid analysis: Asp(1.0), Ser(1.4), Gly(2.9), Cys(1.4), Ile(1.9), Phe(1.9), Arg(1.9).

## Example 29

Seryl-Seryl-Cysteinyl-Phenylalanyl-Glycyl-Glycyl-Isoleucyl-Aspartyl-Arginyl-Isoleucyl-Cysteinyl-Phenylalanyl-Arginyl-Cyclic-Disulfide

0.5 g protected peptide resin, prepared from N-alpha-t-Boc-Arg ($N^G$Tos) Merrifield resin and the amino acids added sequentially in the following order: Boc-Phe, Boc-Cys(4-MeBzl), Boc-Ile, Boc-Arg($N^G$Tos), Boc-Asp(ß-cyclohexyl), Boc-Ile, Boc-Gly, Boc-Gly, Boc-Phe, Boc-Cys(4-MeBzl), Boc-Ser(Bzl), Boc-Ser(Bzl), was treated with HF (liquid) and purified as described in Example 2 yielding a white powder. 27 mg.

$FAB^+$ ($M+H^+$) at M/Z: 1458.

Amino acid analysis: Asp(1.0), Ser(1.5), Gly(2.1), Cys(1.5), Ile(2.0), Phe(2.1), Arg(2.0).

## Example 30

**Seryl-Seryl-Cysteinyl-(D)Phenylalanyl-Glycyl-Glycyl-Arginyl-Isoleucyl-Aspartyl-Arginyl-Isoleucyl-Cysteinyl-Phenylalanyl-Arginyl Cyclic Disulfide**

1.00 g protected peptide resin, prepared from N-alpha-t-Boc-Arg-(Ng-Tos) Merrifield resin and the amino acids added sequentially in the following order: Boc-Phe, Boc-Cys(4-Me-Bzl), Boc-Ile, Boc-Arg($N^G$-Tos), Boc-Asp(ß-cyclohexyl), Boc-Ile, Boc-Arg($N^G$-Tos), Boc-Gly, Boc-Gly, Boc-(D)Phe, Boc-Cys(4-MeBzl), Boc-Ser(Bzl), Boc-Ser(Bzl), was treated with HF (liquid) and purified as described in Example 2. The title compound was obtained as a white powder. 36.0 mg. FAB (M+H) at M/Z: 1615 Amino Acid Analysis: Arg(3.0), Asp(0.9), Cys(1.8), Ile(2.0), Phe(2.0), Ser(1.4), Gly(2.1).
The 300 MHz NMR is consistent with the proposed structure.

## Example 31

**Threonyl-Threonyl-Cysteinyl-Phenylalanyl-Glycyl-Glycyl-Arginyl-Isoleucyl-Aspartyl-Arginyl-Isoleucyl-Cysteinyl-Phenylalanyl-Arginyl Cyclic Disulfide**

0.80 g protect peptide resin, prepared from N-alpha-t-Boc-Arg-($N^G$-Tos) Merrifield resin and the amino acids added sequentially in the following order: Boc-Phe, Boc-Cys(4-Me-Bzl), Boc-Ile, Boc-Arg($N^G$-Tos), Boc-Asp(ß-cyclohexyl), Boc-Ile, Boc-Arg($N^G$-Tos), Boc-Gly, Boc-Gly, Boc-Phe, Boc-Cys(4-MeBzl),

Boc-Thr(Bzl), Boc-Thr(Bzl), was treated with HF (liquid) and purified as described in Example 2. The title compound was obtained as a white powder. 7.0 mg. FAB (M+H) at M/Z: 1643 Amino Acid Analysis: Arg(3.0), Asp(1.0), Cys(1.8), Ile(2.0), Phe(2.1), Gly(2.1), Thr(1.7).
The 300 MHz NMR is consistent with the proposed structure.

Example 32

Seryl-Seryl-Cysteinyl-Phenylalanyl-Glycyl-(D)Alanyl-Arginyl-Isoleucyl-Aspartyl-Arginyl-Isoleucyl-Cysteinyl-Phenylalanyl-Arginyl Cyclic Disulfide

0.80 g protected peptide resin, prepared from N-alpha-t-Boc-Arg-($N^G$-Tos) Merrifield resin and the amino acids added sequentially in the following order: Boc-Phe, Boc-Cys(4-Me-Bzl), Boc-Ile, Boc-Arg($N^G$-Tos), Boc-Asp(ß-cyclohexyl), Boc-Ile, Boc-Arg($N^G$-Tos), Boc-(D)Ala, Boc-Gly, Boc-Phe, Boc-Cys(4-MeBzl), Boc-Ser(Bzl), Boc-Ser(Bzl), was treated with HF (liquid) and purified as described in Example 2. The title compound was obtained as a white powder. 9.0 mg. FAB (M+H) at M/Z: 1629 Amino Acid Analysis: Arg(2.8), Asp(1.2), Cys(0.0), Ile(1.9), Phe(2.0), Ser(2.2), Gly(1.1), Ala(1.0).
The 300 MHz NMR is consistent with the proposed structure.

Example 33

Seryl-Seryl-Cysteinyl-Phenylalanyl-Glycyl-Glycyl-Ornithinyl-Isoleucyl-Aspartyl-Arginyl-Isoleucyl-Cysteinyl-Phenylalanyl-Arginyl Cyclic Disulfide

0.80 g protected peptide resin, prepared from N-alpha-t-Boc-Arg-($N^G$-Tos) Merrifield resin and the amino acids added sequentially in the following order: Boc-Phe, Boc-Cys(4-Me-Bzl), Boc-Ile, Boc-Arg($N^G$-Tos), Boc-Asp(ß-cyclohexyl), Boc-Ile, Boc-Ornithinyl(p-Tos), Boc-Gly, Boc-Gly, Boc-Phe, Boc-Cys(4-MeBzl), Boc-Ser(Bzl), Boc-Ser(Bzl), was treated with HF (liquid and purified as described in Example 2. The title compound was obtained as a white powder. 24.2 mg. Amino Acid Analysis: Arg(1.8), Asp(1.0), Cys(1.7), Ile(1.9), Phe(2.0), Ser(1.6), Gly(2.1). The 300 MHz NMR is consistent with the proposed structure.

Example 34

Seryl-Seryl-Cysteinyl-Phenylalanyl-Glycyl-Glycyl-Arginyl-Isoleucyl-Aspartyl-Arginyl-Isoleucyl-Cysteinyl-Glycyl-Arginyl Cyclic Disulfide

0.80 g protected peptide resin, prepared from N-alpha-t-Boc-Arg-($N^G$-Tos) Merrifield resin and he amino acids added sequentially in the following order: Boc-Glycyl, Boc-Cys(4-Me-Bzl), Boc-Ile, Boc-Arg($N^G$-Tos), Boc-Asp(ß-cyclohexyl), Boc-Ile, Boc-Arg($N^G$-Tos), Boc-Gly, Boc-Gly, Boc-Phe, Boc-Cys(4-MeBzl), Boc-Ser(Bzl), Boc-Ser(Bzl), was treated with HF (liquid) and purified as described in Example 2. The title compound was obtained as a white powder. 9.7 mg. FAB (M+H) at M/Z: 1523.

Amino Acid Analysis: Arg(3.1), Asp(1.1), Cys(1.7), Ile(2.0) Phe(1.1), Ser(1.8), Gly(3.3).
The 300 MHz NMR is consistent with the proposed structure.

## Example 35

### Seryl-Seryl-Cysteinyl-Phenylalanyl-Glycyl-Glycyl-Arginyl-Isoleucyl-Aspartyl-Arginyl-Isoleucyl-Cysteinyl-Phenylalanyl-Lysinyl Cyclic Disulfide

0.75 g protected peptide resin, prepared from Boc-Lys-(Cl-Z) Merrifield resin and the amino acids added sequentially in the following order. Boc-Phe, Boc-Cys(4-MeBzl), Boc-Ile, Boc-Arg($N^G$-Tos), Boc-Asp(ß-cyclohexyl), Boc-Ile, Boc-Arg($N^G$-Tos), Boc-Gly, Boc-Gly, Boc-Phe, Boc-Cys(4-MeBzl), Boc-Ser(Bzl), Boc-Ser(Bzl), was treated with HF (liquid) and purified as described in Example 2. The title compound was obtained as a white powder. 21.0 mg. FAB (M+H) at M/Z: 1587.
Amino Acid Analysis: Arg(2.0), Asp(1.0), Cys(1.8), Ile(1.9), Phe(2.0), Ser(1.7), Gly(2.0), Lys(1.0).
The 300 MHz NMR is consistent with the proposed structure.

## Example 36

### Seryl-Seryl-Cysteinyl-Phenylalanyl-Betaalanyl-Arginyl-Isoleucyl-Aspartyl-Arginyl-Isoleucyl-Cysteinyl-Phenylalanyl-Arginyl Cyclic Disulfide

0.85 g protected peptide resin, prepared from N-alpha-t-Boc-Arg-($N^G$-Tos) Merrifield resin and the amino acids added sequentially in the following order: Boc-Phe, Boc-Cys(4-MeBzl), Boc-Ile, Boc-Arg($N^G$-Tos),

Boc-Asp(ß-cyclohexyl), Boc-Ile, Boc-Arg($N^G$)-Tos, Boc-BetaAla, Boc-Phe, Boc-Cys(4-MeBzl), Boc-Ser(Bzl), Boc-Ser(Bzl), was treated with HF (liquid) and purified as described in Example 2. The title compound was obtained as a white powder. 18.0 mg. FAB (M+H) at M/Z: 1572.
Amino Acid Analysis: Arg(3.0), Asp(1.0), Cys(1.9), Ile(2.0), Phe(2.1), Ser(1.8).
The 300 MHz NMR is consistent with the proposed structure.

## Example 37

### Seryl-Seryl-Cysteinyl-Phenylalanyl-Glycyl-Glycyl-Arginyl-Isoleucyl-Aspartyl-Arginyl-Isoleucyl-Cysteinyl-Phenylalanyl-Arginyl-Tyrosinyl Cyclic Disulfide

0.50 g protected peptide resin, prepared from Boc-Tyr-(Br-Z) Merrifield resin and the amino acids added sequentially in the following order: Boc-Arg($N^G$-Tos), Boc-Phe, Boc-Cys(4-MeBzl), Boc-Ile, Boc-Arg($N^G$-Tos), Boc-Asp(ß-cyclohexyl), Boc-Ile, Boc-Arg($N^G$-Tos), Boc-Gly, Boc-Gly, Boc-Phe, Boc-Cys(4-MeBzl), Boc-Ser(Bzl), Boc-Ser(Bzl), was treated with HF (liquid) and purified as described in Example 2. The title compound was obtained as a white powder 32.3 mg. FAB (M+H) at M/Z: 1778.
Amino Acid Analysis: Arg(3.1), Asp(1.1), Cys(1.9), Gly(2.1), Ile(2.0), Phe(2.0), Ser(1.8), Tyr(0.9).
The 300 MHz NMR is consistent with the proposed structure.

Example 38

Seryl-Seryl-Cysteinyl-Cyclohexylalanyl-Glycyl-Glycyl-Arginyl-Isoleucyl-Aspartyl-Arginyl-Isoleucyl-Cysteinyl-Phenylalanyl-Arginyl Cyclic Disulfide

0.50 g protected peptide resin, prepared from N-alpha-t-Boc-Arg-($N^G$-Tos) Merrifield resin and the amino acids added sequentially in the following order: Boc-Phe, Boc-Cys(4-MeBzl), Boc-Ile, Boc-Arg($N^G$-Tos), Boc-Asp(ß-cyclohexyl), Boc-Ile, Boc-Arg($N^G$-Tos), Boc-Gly, Boc-Gly, Boc-Cyclohexylalanyl, Boc-Cys(4-MeBzl), Boc-Ser(Bzl), Boc-Ser(Bzl), was treated with HF (liquid) and purified as described in Example 2. The title compound was obtained as a white powder. 15.8 mg. FAB (M+H) at M/Z: 1620.
Amino Acid Analysis: Arg(2.8), Asp(1.0), Cys(1.8), Gly(1.9), Ile(2.0), Phe(1.0), Ser(1.6).
The 300 MHz NMR is consistent with the proposed structure.

Example 39

Seryl-Seryl-Cysteinyl-Phenylalanyl-Glycyl-Glycyl-Arginyl-Isoleucyl-Aspartyl-Arginyl-Phenylalanyl-Cysteinyl-Phenylalanyl-Arginyl Cyclic Disulfide

0.50 g protected peptide resin, prepared from N-alpha-t-Boc-Arg-($N^G$-Tos) Merrifield resin and the amino acids added sequentially in the following order: Boc-Phe, Boc-Cys(4-MeBzl), Boc-Phe, Boc-Arg($N^G$-Tos), Boc-Asp(ß-cyclohexyl), Boc-Ile, Boc-Arg($N^G$-Tos), Boc-Gly, Boc-Gly, Boc-Phe, Boc-Cys(4-MeBzl), Boc-Ser(Bzl), Boc-Ser(Bzl), was treated with HF (liquid)

and purified as described in Example 2. The title compound was obtained as a white powder. 30.5 mg. FAB (M+H) at M/Z: 1649.
Amino Acid Analysis: Arg(3.0), Asp(1.0), Cys(1.7), Gly(2.1), Ile(1.0), Phe(2.9), Ser(1.8).
The 300 MHz NMR is consistent with the proposed structure.

## Example 40

**Seryl-Seryl-Cysteinyl-Phenylalanyl-Glycyl-Glycyl-Arginyl-Isoleucyl-Aspartyl-(D)-Arginyl-Isoleucyl-Cysteinyl-Phenylalanyl-Arginyl Cyclic Disulfide**

0.45 g protected peptide resin, prepared from N-alpha-t-Boc-Arg-($N^G$-Tos)Merrifield resin and the amino acids added sequentially in the following order: Boc-Phe, Boc-Cys(4-MeBzl), Boc-Ile, Boc-Arg($N^G$-Tos), Boc-Asp(ß-cyclohexyl), Boc-Ile, Boc-(D)-Arg($N^G$-Tos), Boc-Gly, Boc-Gly, Boc-Phe, Boc-Cys(4-MeBzl), Boc-Ser(Bzl), Boc-Ser(Bzl), was treated with HF (liquid) and purified as described in Example 2. The title compound was obtained as a white powder. 28 mg. FAB (M+H) at M/Z: 1615.
Amino Acid Analysis: Arg(3.1), Asp(1.0), Cys(1.6), Gly(2.2) Ile(1.9), Phe(1.8), Ser(1.5).
The 300 MHz NMR is consistent with the proposed structure.

## Example 41

Seryl-Seryl-Cysteinyl-Phenylalanyl-Glycyl-Glycyl-(D)-Arginyl-Isoleucyl-Aspartyl-Arginyl-Isoleucyl-Cysteinyl-Phenylalanyl-Arginyl Cyclic Disulfide

0.45 g protected peptide resin, prepared from N-alpha-t-Boc-Arg-($N^G$-Tos)Merrifield resin and the amino acids added sequentially in the following order: Boc-Phe, Boc-Cys(4-MeBzl), Boc-Ile, Boc-(D)-Arg($N^G$-Tos), Boc-Asp(ß-cyclohexyl), Boc-Ile, Boc-Arg($N^G$-Tos), Boc-Gly, Boc-Gly, Boc-Phe, Boc-Cys(4-MeBzl), Boc-Ser(Bzl), Boc-Ser(Bzl), was treated with HF (liquid) and purified as described in Example 2. The title compound was obtained as a white powder. 25 mg. FAB (M+H) at M/Z: 1615.
Amino Acid Analysis: Arg(2.8), Asp(1.0), Cys(1.5), Gly(2.1), Ile(1.9), Phe(1.9), Ser(1.5).
The 300 MHz NMR is consistent with the proposed structure.

## Example 42

Seryl-Seryl-Cysteinyl-Phenylalanyl-Glycyl-Glycyl-Arginyl-Isoleucyl-Aspartyl-Arginyl-Isoleucyl-Cysteinyl-Cyclohexylalanyl-Arginyl Cyclic Disulfide

0.45 g protected peptide resin, prepared from N-alpha-t-Boc-Arg-($N^G$-Tos) Merrifield resin and the amino acids added sequentially in the following order: Boc-Cha, Boc-Cys(4-MeBzl), Boc-Ile, Boc-Arg($N^G$-Tos), Boc-Asp(ß-cyclohexyl), Boc-Ile, Boc-Arg($N^G$-Tos), Boc-Gly, Boc-Gly, Boc-Phe, Boc-(D)-Alanine, Boc-Cys(4-MeBzl), Boc-Ser(Bzl), Boc-Ser(Bzl, was treated with HF (liquid) and purified as described in Example

2. The title compound was obtained as a white powder. 40 mg. FAB (M+H) at M/Z: 1621.
Amino Acid Analysis: Arg(2.9), Asp(1.0), Cys(1.6), Gly(1.9), Ile(1.9), Phe(1.0), Ser(1.5).
The 300 MHz NMR is consistent with the proposed structure.

Example 43

D-Arginyl-Cysteinyl-Phenylalanyl-Glycyl-Glycyl-Arginyl-Isoleucyl-Aspartyl-Arginyl-Isoleucyl-Cysteinyl-Phenylalanyl-Arginyl Cyclic Dislulfide

0.45 g protected peptide resin, prepared from N-alpha-t-Boc-Arg-($N^G$-Tos) merrifield resin and the amino acids added sequentially in the following order: Boc-Phe, Boc-Cys(4-MeBzl), Boc-Ile, Boc-Arg($N^G$-Tos), Boc-Asp(beta-cyclohexyl), Boc-Ile, Boc-Arg($N^G$-Tos), Boc-Gly, Boc-Gly, Boc-Phe, Boc-Cys(4-MeBzl), Boc-(D)-Arg($N^G$-Tos), was treated with HF (liquid) and purified as described in Example 2. The title compound was obtained as a white powder. 8 mg.
FAB (M+H) at M/Z: 1597.
Amino Acid Analysis: Arg (3.9), Asp (1.0), Cys (2.0), Gly (2.4), Ile (2.0), Phe (2.0).
The 300 MHz NMR is consistent with the proposed structure.

Example 44

Arginyl-Cysteinyl-Phenylalanyl-Glycyl-Glycyl-Arginyl-Isoleucyl-Aspartyl-Arginyl-Isoleucyl-Cysteinyl-Phenylalanyl-Arginyl Cyclic Disulfide

0.45 g protected peptide resin, prepared from N-alpha-t-Boc-Arg-($N^G$-Tos) Merrifield resin and the amino acids added sequentially in the following order: Boc-Phe, Boc-Cys(4-MeBzl), Boc-Ile, Boc-Arg($N^G$-Tos), Boc-Asp(ß-cyclohexyl), Boc-Ile, Boc-Arg($N^G$-Tos), Boc-Gly, Boc-Gly, Boc-Phe, Boc-Cys(4-MeBzl), Boc-Arg($N^G$-Tos), was treated with HF (liquid) and purified as described in Example 2. The title compound was obtained as a white powder. 39 mg. FAB (M+H) at M/Z: 1597.

Amino Acid Analysis: Arg(3.9), Asp(1.0), Cys(1.8), Gly(2.0),Ile(1.9), Phe(1.9).

The 300 MHz NMR is consistent with the proposed structure.

Example 45

Seryl-Seryl-Cysteinyl-Phenylalanyl-Glycyl-Glycyl-Arginyl-Cyclohexylalanyl-Aspartyl-Arginyl-Isoleucyl-Cysteinyl-Phenylalanyl-Arginyl Cyclic Disulfide

0.45 g protected peptide resin, prepared from N-alpha-t-Boc-Arg-($N^G$-Tos) Merrifield resin and the amino acids added sequentially in the following order: Boc-Phe, Boc-Cys(4-MeBzl), Boc-Ile, Boc-Arg($N^G$-Tos), Boc-Asp(ß-cyclohexyl), Boc-Cha, Boc-Arg($N^G$-Tos), Boc-Gly, Boc-Gly, Boc-Phe, Boc-(D)-Alanine,

Boc-Cys(4-MeBzl), Boc-Ser(Bzl), Boc-Ser(Bzl), was treated with HF (liquid) and purified as described in Example 2. The title compound was obtained as a white powder. 25 mg. FAB (M+H) at M/Z: 1655.
Amino Acid Analysis: Arg(3.0), Asp(1.0), Cys(1.6), Gly(2.0), Ile(0.9) Phe(1.8), Ser(1.5).
The 300 MHz NMR is consistent with the proposed structure.

## Example 46

### Arginyl-Cysteinyl-Phenylalanyl-Glycyl-Glycyl-Arginyl-Isoleucyl-Glycyl-Glycyl-Isoleucyl-Cysteinyl-Phenylalanyl-Arginyl Cyclic Disulfide

0.45 g protected peptide resin, prepared from N-alpha-t-Boc-Arg-($N^G$-Tos) Merrifield resin and the amino acids added sequentially in the following order: Boc-Phe, Boc-Cys(4-MeBzl), Boc-Ile, Boc-Glycine, Boc-Glycine, Boc-Ile, Boc-Arg($N^G$-Tos), Boc-Gly, Boc-Gly, Boc-Phe, Boc-Cys(4-MeBzl), Boc-Arg($N^G$-Tos), was treated with HF (liquid) and purified as described in Example 2. The title compound was obtained as a white powder. 52 mg. FAB (M+H) at M/Z: 1439.
Amino Acid Analysis: Arg(3.0), Cys(1.8), Gly(4.1), Ile(2.0), Phe(2.0).
The 300 MHz NMR is consistent with the proposed structure.

## Example 47

### Arginyl-Cysteinyl-Phenylalanyl-Glycyl-Glycyl-Arginyl-Isoleucyl-Aspartyl-Arginyl-Isoleucyl-Cysteinyl-Cyclohexylalanyl-Arginyl Cyclic Disulfide

0.69 g protected peptide resin, prepared from N-alpha-t-Boc-Arg-($N^G$-Tos) Merrifield resin and the amino acids added sequentially in the following order: Boc-Cha, Boc-Cys(4-MeBzl), Boc-Ile, Boc-Arg($N^G$-Tos), Boc-Asp(ß-cyclohexyl), Boc-Ile, Boc-Arg($N^G$-Tos), Boc-Gly, Boc-Gly, Boc-Phe, Boc-Cys(4-MeBzl), Boc-Arg($N^G$-Tos) was treated with HF (liquid) and purified as described in Example 2. The title compound was obtained as a white powder. 15 mg. FAB (M+H) at M/Z: 1602.

Amino Acid Analysis: Arg(4.0), Asp(1.1), Cys(1.9), Gly(2.2), Ile(1.8), Phe(1.1).

The 300 MHz $^1H$ NMR is consistent with the proposed structure.

## Example 48

### Arginyl-Cysteinyl-Phenylalanyl-Glycyl-Glycyl-Arginyl-Isoleucyl-Aspartyl-Arginyl-Isoleucyl-Cysteinyl-Arginyl Cyclic Disulfide

0.40 g protected peptide resin, prepared from N-alpha-t-Boc-Arg-($N^G$-Tos) Merrifield resin and the amino acids added sequentially in the following order: Boc-Cys(4-MeBzl), Boc-Ile, Boc-Arg($N^G$-Tos), Boc-Asp(ß-cyclohexyl), Boc-Ile, Boc-Arg($N^G$-Tos), Boc-Gly, Boc-Gly, Boc-Phe, Boc-Cys(4-MeBzl), Boc-Arg($N^G$-Tos) was treated with HF (liquid) and purified as described in Example 2. The title compound was obtained as a white powder. 16 mg. FAB (M+H) at

M/Z: 1449.

Amino Acid Analysis: Arg(4.0), Asp(0.9), Cys(1.7), Gly(2.0), Ile(2.0), Phe(1.0).

The 300 MHz $^{1}H$ NMR is consistent with the proposed structure.

## Example 49

### Seryl-Seryl-Cysteinyl-D-Alanyl-Glycyl-Glycyl-Arginyl-Isoleucyl-Aspartyl-Arginyl-Isoleucyl-Cysteinyl-Phenylalanyl-Arginyl Cyclic Disulfide

0.40 g protected peptide resin, prepared from N-alpha-t-Boc-Arg($N^G$-Tos) Merrifield resin and the amino acids added sequentially in the following order: Boc-Phe, Boc-Cys(4-MeBzl), Boc-Ile, Boc-Arg ($N^G$-Tos), Boc-Asp(ß-cyclohexyl), Boc-Ile, Boc-Arg ($N^G$-Tos), Boc-Gly, Boc-Gly, Boc-D-Ala, Boc-Cys(4-MeBzl), Boc-Ser(Bzl), Boc-Ser(Bzl), was treated with HF (liquid) and purified as described in Example 2. The title compound was obtained as a white powder. 24 mg. FAB (M+H) at M/Z: 1538.

Amino Acid Analysis: Ala(1.0), Arg(3.0), Asp(1.1), Cys(1.7), Gly(1.9), Ile(2.0), Phe(1.0), Ser(1.6).

The 300 MHZ $^{1}H$ NMR is consistent with the proposed structure.

## Example 50

### Arginyl-Cysteinyl-Phenylalanyl-Glycyl-Glycyl-Arginyl-Isoleucyl-Aspartyl-Arginyl-Isoleucyl-Cysteinyl-Cyclic Disulfide

0.40 g protected peptide resin, prepared from N-alpha-t-Boc-Arg-($N^G$-Tos) Merrifield resin and the

amino acids added sequentially in the following order: Boc-Cys(4-MeBzl), Boc-Ile, Boc-Arg($N^G$-Tos), Boc-Asp(ß-cyclohexyl), Boc-Ile, Boc-Arg($N^G$-Tos), Boc-Gly, Boc-Gly, Boc-Phe, Boc-Cys(4-MeBzl), Boc-Arg($N^G$-Tos) was treated with HF (liquid) and purified as described in Example 2. The title compound was obtained as a white powder. 31 mg. FAB (M+H) at M/Z: 1293.

Amino Acid Analysis: Arg(3.0), Asp(1.1), Cys(1.7), Gly(2.1), Ile(2.0), Phe(1.0).

The 300 MHz $^1$H NMR is consistent with the proposed structure.

## Example 51

### 7-Amino-heptanoyl-Cysteinyl-Phenylalanyl-Glycyl Glycyl-Arginyl-Isoleucyl-Aspartyl-Arginyl-Isoleucyl-Cysteinyl-Phenylalanyl-Arginyl Cyclic Disulfide

0.45 g protected peptide resin, prepared from N-alpha-t-Boc-Arg($N^G$-Tos) Merrifield resin and the amino acids added sequentially in the following order: Boc-Phe, Boc-Cys(4-MeBzl), Boc-Ile, Boc-Arg($N^G$-Tos), Boc-Asp(ß-cyclohexyl), Boc-Ile, Boc-Arg($N^G$-Tos), Boc-Gly, Boc-Gly, Boc-Phe, Boc-Cys(4-MeBzl),Boc-7-amino heptanoic acid was treated with HF (liquid) and purified as described in Example 2. The title compound was obtained as a white powder. 16 mg.

$FAB^+$ $(M+H)^+$ at M/Z: 1567.

Amino Acid Analysis: Arg(3.0), Asp(1.1), Cys(1.2), Gly(2.4), Ile(1.5), Phe(2.0).

The 300 MHZ $^1$H NMR is consistent with the proposed structure.

Example 52

8-Aminooctanoyl-Cysteinyl-Phenylalanyl-Glycyl-Glycyl-Arginyl-Isoleucyl-Aspartyl-Arginyl-Isoleucyl-Cysteinyl-Phenylalanyl-Arginyl Cyclic Disulfide

0.50 g protected peptide resin, prepared from Boc-Arg($N^G$-Tosyl)-O-resin and the amino acids added sequentially in the following order: Boc-Phe, Boc-Cys(MeBzl), Boc-Ile, Boc-Arg($N^G$-Tos), Boc-Asp(ß-cyclohexyl), Boc-Leu, Boc-Arg($N^G$-Tos), Boc-Gly, Boc-Gly, Boc-Phe, Boc-Cys(4-MeBzl),Boc-8-Aminooctanoic acid was treated with HF (liquid) and purified as described in Example 2. The title compound was obtained as a white powder. 20 mg.

$FAB^+$ $(M+H)^+$ at M/Z: 1583.

Amino Acid Analysis: Arg(3.2), Asp(1.0), Cys(1.8), Gly(2.1), Ile(1.9), Phe(1.9).

The 300 MHZ $^1H$ NMR is consistent with the proposed structure.

Example 53

5-Aminopentanoyl-Cysteinyl-Phenylalanyl-Glycyl-Glycyl-Arginyl-Isoleucyl-Aspartyl-Arginyl-Isoleucyl-Cysteinyl-Phenylalanyl-Arginyl Cyclic Disulfide

0.50 g protected peptide resin, prepared from Boc-Arg($N^G$-Tosyl)-O-resin and the amino acids added sequentially in the following order: Boc-Phe, Boc-Cys(MeBzl), Boc-Ile, Boc-Arg($N^G$-Tos), Boc-Asp(ß-cyclohexyl), Boc-Ile, Boc-Arg($N^G$-Tos), Boc-Gly, Boc-Gly, Boc-Phe, Boc-Cys(4-MeBzl), Boc-5-Aminopentanoic acid, was treated with HF (liquid)

and purified as described in Example 2. The title compound was obtained as a white powder. 10 mg.
$FAB^+$ $(M+H)^+$ at M/Z: 1539.
Amino Acid Analysis: Arg(3.2), Asp(1.0), Cys(1.8), Gly(2.1), Ile(1.9), Phe(1.9).
The 300 MHZ $^1H$ NMR is consistent with the proposed structure.

## Example 54

### Seryl-Seryl-Cysteinyl-Phenylalanyl-Glycyl Glycyl-Arginyl-Isoleucyl-Aspartyl-Arginyl-Isoleucyl-Cysteinyl-Prolyl-Arginyl Cyclic Disulfide

0.50 g protected peptide resin, prepared from Boc-Arg($N^G$-Tosyl)-O-resin and the amino acids added sequentially in the following order: Boc-Pro, Boc-Cys(MeBzl), Boc-Ile, Boc-Arg($N^G$-Tos), Boc-Asp(ß-cyclohexyl), Boc-Leu, Boc-Arg($N^G$-Tos), Boc-Gly, Boc-Gly, Boc-Phe, Boc-Cys(4-MeBzl), Boc-Ser(Bzl), Boc-Ser(Bzl), was treated with HF (liquid) and purified as described in Example 2. The title compound was obtained as a white powder. 10 mg.
$FAB^+$ $(M+H)^+$ at M/Z: 1565.
Amino Acid Analysis: Arg(2.2), Asp(1.0), Cys(1.8), Gly(2.1), Ile(1.9), Phe(0.9), Ser(1.4), Pro(0.9).
The 300 MHZ $^1H$ NMR is consistent with the proposed structure.

## Example 55

**Seryl-Seryl-Cysteinyl-Phenylalanyl-Glycyl Glycyl-Arginyl-Isoleucyl-Aspartyl-Arginyl-Isoleucyl-Cysteinyl-Seryl-Phenylalanyl-Arginyl Cyclic Disulfide**

0.50 g protected peptide resin, prepared from Boc-Arg($N^G$-Tosyl)-O-resin and the amino acids added sequentially in the following order: Boc-Phe, Boc-Ser(Bzl), Boc-Cys(MeBzl), Boc-Ile, Boc-Arg($N^G$-Tos), Boc-Asp(ß-cyclohexyl), Boc-Ile, Boc-Arg($N^G$-Tos), Boc-Gly, Boc-Gly, Boc-Phe, Boc-Cys(4-MeBzl), Boc-Ser(Bzl), Boc-Ser(Bzl), was treated with HF (liquid) and purified as described in Example 2. The title compound was obtained as a white powder. 10 mg.

$FAB^+$ $(M+H)^+$ at M/Z: 1703.

Amino Acid Analysis: Arg(3.2), Asp(1.0), Cys(1.8), Gly(2.2), Ile(2.0), Phe(0.9), Ser(2.4).

The 300 MHZ $^1H$ NMR is consistent with the proposed structure.

## Example 56

**Seryl-Seryl-Cysteinyl-Phenylalanyl-Glycyl Glycyl-Arginyl-Isoleucyl-Aspartyl-Arginyl-Isoleucyl-Cysteinyl-8-Aminooctanoyl-Arginyl Cyclic Disulfide**

0.50 g protected peptide resin, prepared from Boc-Arg($N^G$-Tosyl)-O-resin and the amino acids added sequentially in the following order: Boc-8-Aminooctanoic acid, Boc-Cys(MeBzl), Boc-Ile, Boc-Arg($N^G$-Tos), Boc-Asp(ß-cyclohexyl), Boc-Ile, Boc-Arg($N^G$-Tos), Boc-Gly, Boc-Gly, Boc-Phe,

Boc-Cys(4-MeBzl), Boc-Ser(Bzl), Boc-Ser(Bzl), was treated with HF (liquid) and purified as described in Example 2. The title compound was obtained as a white powder. 10 mg.

$FAB^+$ $(M+H)^+$ at M/Z: 1609.

Amino Acid Analysis: Arg(3.2), Asp(1.0), Cys(1.8), Gly(2.2), Ile(2.0), Phe(0.9), Ser(1.4).

The 300 MHZ $^1H$ NMR is consistent with the proposed structure.

## Example 57

### Seryl-Seryl-Cysteinyl-Phenylalanyl-Glycyl Glycyl-Arginyl-Isoleucyl-Aspartyl-Arginyl-Isoleucyl-Cysteinyl-Tetrahydroisoquin -3-oyl-Arginyl Cyclic Disulfide

0.50 g protected peptide resin, prepared from Boc-Arg($N^G$-Tosyl)-O-resin and the amino acids added sequentially in the following order: Boc-Tic, Boc-Cys(MeBzl), Boc-Ile, Boc-Arg($N^G$-Tos), Boc-Asp(ß-cyclohexyl), Boc-Leu, Boc-Arg($N^G$-Tos), Boc-Gly, Boc-Gly, Boc-Phe, Boc-Cys(4-MeBzl), Boc-Ser(Bzl), Boc-Ser(Bzl), was treated with HF (liquid) and purified as described in Example 2. The title compound was obtained as a white powder. 10 mg.

Amino Acid Analysis: Arg(2.2), Asp(1.0), Cys(1.8), Gly(2.2), Ile(1.9), Phe(0.9), Ser(1.4).

The 300 MHZ $^1H$ NMR is consistent with the proposed structure.

Example 58

Seryl-Seryl-Cysteinyl-Tetrahydroisoquin-3-oyl-Glycyl Glycyl-Arginyl-Isoleucyl-Aspartyl-Arginyl-Isoleucyl-Cysteinyl-Phenylalanyl-Arginyl Cyclic Disulfide

0.50 g protected peptide resin, prepared from Boc-Arg($N^G$-Tosyl)-O-resin and the amino acids added sequentially in the following order: Boc-Phe, Boc-Cys(MeBzl), Boc-Ile, Boc-Arg($N^G$-Tos), Boc-Asp(ß-cyclohexyl), Boc-Leu, Boc-Arg($N^G$-Tos), Boc-Gly, Boc-Gly, Boc-Tic, Boc-Cys(4-MeBzl), Boc-Ser(Bzl), Boc-Ser(Bzl), was treated with HF (liquid) and purified as described in Example 2. The title compound was obtained as a white powder. 10 mg.
$FAB^+$ $(M+H)^+$ at M/Z: 1626.
Amino Acid Analysis: Arg(3.2), Asp(1.0), Cys(1.8), Gly(2.2), Ile(1.9), Phe(0.9), Ser(1.4).
The 300 MHZ $^1H$ NMR is consistent with the proposed structure.

Example 59

Seryl-Seryl-Cysteinyl-Phenylalanyl-Glycyl Glycyl-Arginyl-Isoleucyl-Aspartyl-Ornithinyl-Isoleucyl-Cysteinyl-Phenylalanyl-Arginyl Cyclic Disulfide

0.50 g protected peptide resin, prepared from Boc-Arg($N^G$-Tosyl)-O-resin and the amino acids added sequentially in the following order: Boc-Phe, Boc-Cys(MeBzl), Boc-Ile, Boc-Orn(N-Tos), Boc-Asp(ß-cyclohexyl), Boc-Leu, Boc-Arg($N^G$-Tos), Boc-Gly, Boc-Gly, Boc-Phe, Boc-Cys(4-MeBzl), Boc-Ser(Bzl), Boc-Ser(Bzl), was treated with HF (liquid)

and purified as described in Example 2. The title compound was obtained as a white powder. 10 mg.
FAB $^{+}$ $(M+H)^{+}$ at M/Z: 1572.
Amino Acid Analysis: Arg(2.2), Asp(1.0), Cys(1.8), Gly(2.2), Ile(1.9), Phe(1.9), Ser(1.4).
The 300 MHZ $^{1}$H NMR is consistent with the proposed structure.

Example 60

Seryl-Seryl-Cysteinyl-Phenylalanyl-Glycyl Glycyl-Arginyl-Isoleucyl-Cysteinyl-Phenylalanyl-Arginyl Cyclic Disulfide

0.50 g protected peptide resin, prepared from Boc-Arg($N^{G}$-Tosyl)-O-resin and the amino acids added sequentially in the following order: Boc-Phe, Boc-Cys(MeBzl), Boc-Ile, Boc-Arg($N^{G}$-Tos), Boc-Asp(ß-cyclohexyl), Boc-Leu, Boc-Arg($N^{G}$-Tos), Boc-Gly, Boc-Gly, Boc-Phe, Boc-Cys(4-MeBzl), Boc-Ser(Bzl), Boc-Ser(Bzl), was treated with HF (liquid) and purified as described in Example 2. The title compound was obtained as a white powder. 10 mg.
FAB $^{+}$ $(M+H)^{+}$ at M/Z: 1615.
Amino Acid Analysis: Arg(3.2), Asp(1.0), Cys(1.8), Gly(2.2), Ile(1.1), Leu(1.0), Phe(1.8), Ser(1.4).
The 300 MHZ $^{1}$H NMR is consistent with the proposed structure.

Example 61

Seryl-Seryl-Cysteinyl-Phenylalanyl-Glycyl Glycyl-Arginyl-Propargylglycyl-Aspartyl-Arginyl-Isoleucyl-Cysteinyl-Phenylalanyl-Arginyl Cyclic Disulfide

0.50 g protected peptide resin, prepared from Boc-Arg($N^G$-Tosyl)-O-resin and the amino acids added sequentially in the following order: Boc-Phe, Boc-Cys(MeBzl), Boc-Ile, Boc-Arg($N^G$-Tos), Boc-Asp(ß-cyclohexyl), Boc-PrGly, Boc-Arg($N^G$-Tos), Boc-Gly, Boc-Gly, Boc-Phe, Boc-Cys(4-MeBzl), Boc-Ser(Bzl), Boc-Ser(Bzl), was treated with HF (liquid) and purified as described in Example 2. The title compound was obtained as a white powder. 10 mg.
$FAB^+$ $(M+H)^+$ at M/Z: 1596.
Amino Acid Analysis: Arg(3.2), Asp(1.0), Cys(1.8), Gly(2.2), Ile(1.1), Phe(0.9), Ser(1.4).
The 300 MHZ $^1H$ NMR is consistent with the proposed structure.

Example 62

Seryl-Seryl-Cysteinyl-Phenylalanyl-Meta-aminomethybenzoyl-Arginyl-Isoleucyl-Aspartyl-Arginyl-Isoleucyl-Cysteinyl-Phenylalanyl-Arginyl Cyclic Disulfide

0.50 g protected peptide resin, prepared from Boc-Arg($N^G$-Tosyl)-O-resin and the amino acids added sequentially in the following order: Boc-Phe, Boc-Cys(MeBzl), Boc-Ile, Boc-Arg($N^G$-Tos), Boc-Asp(ß-cyclohexyl), Boc-Ile, Boc-Arg($N^G$-Tos), Boc-meta-Aminomethylbenzoic acid, Boc-Phe,

Boc-Cys(4-MeBzl), Boc-Ser(Bzl), Boc-Ser(Bzl), was treated with HF (liquid) and purified as described in Example 2. The title compound was obtained as a white powder. 10 mg.

FAB $^+$ (M+H) $^+$ at M/Z: 1634.

Amino Acid Analysis: Arg(2.2), Asp(1.0), Cys(1.8), Ile(1.9), Phe(1.9), Ser(1.4).

The 300 MHZ $^1$H NMR is consistent with the proposed structure.

Example 63

Seryl-Seryl-Cysteinyl-Phenylalanyl-Glycyl-Glycyl-Arginyl-Isoleucyl-Aspartyl-Arginyl-Isoleucyl-Cysteinyl-Asparaginyl-Seryl-Phenylalanyl-Cyclic Disulfide

0.50 g protected peptide resin, prepared from Boc-Phe-O-resin and the amino acids added sequentially in the following order: Boc-Ser(Bzl), Boc-Asn, Boc-Cys(MeBzl), Boc-Ile, Boc-Arg($N^G$-Tos), Boc-Asp(ß-cyclohexyl), Boc-Ile, Boc-Arg($N^G$-Tos), Boc-Gly, Boc-Gly, Boc-Phe, Boc-Cys(4-MeBzl), Boc-Ser(Bzl), Boc-Ser(Bzl), was treated with HF (liquid) and purified as described in Example 2. The title compound was obtained as a white powder. 10 mg.

FAB $^+$ (M+H) $^+$ at M/Z: 1660.

Amino Acid Analysis: Arg(2.1), Asp(1.8), Cys(1.8), Gly(2.2), Ile(2.0), Phe(1.9), Ser(2.4).

The 300 MHZ $^1$H NMR is consistent with the proposed structure.

Example 64

Seryl-Seryl-Cysteinyl-Phenylalanyl-Glycyl-Glycyl-Arginyl-Isoleucyl-Aspartyl-Arginyl-Isoleucyl-Cysteinyl-Asparaginyl-Seryl-Cyclic Disulfide

0.50 g protected peptide resin, prepared from Boc-Ser(Bzl)-O-resin and the amino acids added sequentially in the following order: Boc-Asn, Boc-Cys(MeBzl), Boc-Ile, Boc-Arg($N^G$-Tos), Boc-Asp(ß-cyclohexyl), Boc-Ile, Boc-Arg($N^G$-Tos), Boc-Gly, Boc-Gly, Boc-Phe, Boc-Cys(4-MeBzl), Boc-Ser(Bzl), Boc-Ser(Bzl), was treated with HF (liquid) and purified as described in Example 2. The title compound was obtained as a white powder. 27 mg.
FAB $^+$ (M+H) $^+$ at M/Z: 1513.
Amino Acid Analysis: Arg(2.1), Asp(1.8), Cys(1.8), Gly(2.2), Ile(2.0), Phe(0.9), Ser(2.4).
The 300 MHZ $^1$H NMR is consistent with the proposed structure.

Example 65

Seryl-Seryl-Cysteinyl-Phenylalanyl-6-Aminocaproyl-Arginyl-Isoleucyl-Aspartyl-Arginyl-Isoleucyl-Cysteinyl-Phenylalanyl-Arginyl-Cyclic Disulfide

0.50 g protected peptide resin, prepared from Boc-Arg($N^G$-Tosyl)-O-resin and the amino acids added sequentially in the following order: Boc-Phe, Boc-Cys(MeBzl), Boc-Ile, Boc-Arg($N^G$-Tos), Boc-Asp(ß-cyclohexyl), Boc-Ile, Boc-Arg($N^G$-Tos), Boc-6-Aminocaproic acid, Boc-Phe, Boc-Cys(4-MeBzl),

Boc-Ser(Bzl), Boc-Ser(Bzl), was treated with HF (liquid) and purified as described in Example 2. The title compound was obtained as a white powder. 10 mg.
FAB $^{+}$ $(M+H)^{+}$ at M/Z: 1614.
Amino Acid Analysis: Arg(3.1), Asp(1.0), Cys(1.8), Ile(2.0), Phe(1.9), Ser(1.4).
The 300 MHZ $^{1}H$ NMR is consistent with the proposed structure.

Example 66

Vasorelaxant Activity

Female New Zealand white rabbits (2-3 kg) were anesthetized with phenobarbital sodium (30 mg/kg), and the descending thoracic aorta removed. The aorta was immediately placed in physiological saline (PSS) of the following millimolar composition: NaCl 119, KCL 4.7, $CaCl_2$2.5, MgSO4 1.5, NaHC03 25, $KH_2P0_4$ 1.2, dextrose 11, $Na_2$EDTA 0.03. After removal of surrounding tissue, rings 3 mm in length were mounted vertically in 2 ml tissue baths, and attached to Harvard 9529 transducers with 1.5 g resting tension in PSS at 37°C. Rings were equilibrated 15 minutes, exposed to (-)norepinephrine, $1 \times 10^{-5}$M, for 2 minutes, then washed with PSS. After a one hour interval of equilibration with PSS washes every 15 minutes, the tissues were contracted with histamine, $6 \times 10^{-6}$M. Cumulative additions of six half-log concentration increments of test compounds to each bath were begun 30 minutes after histamine. Dilutions of test compounds were made in 50mM sodium phosphate pH 7.4 containing 0.1 bovine serum albumin (vehicle). In each study, the

vasorelaxant responses to vehicle and to the alpha-hANP were observed in control tissues. The negative log of the concentration which produced half-maximal relaxation (pD2) and the maximum vasorelaxant effect (Emax) relative to alpha-hANP are reported. The results of the testing are shown in Table I.

The foregoing is merely illustrative of the invention and is not intended to limit the invention to the disclosed compounds. Variations and changes which are obvious to one skilled in the art are intended to be within the scope and nature of the invention which are defined in the appended claims.

TABLE I

Vasorelaxant Activity

| Example No. | Agonist Testing (mean pD2) |
|---|---|
| 2 | 6.4 |
| 3 | 5.7 |
| 4 | 5.0 |
| 5 | 5.0 |
| 6 | 5.0 |
| 7 | 5.0 |
| 8 | 5.4 |
| 9 | 5.0 |
| 10 | 5.5 |
| 11 | 5.1 |
| 12 | 5.3 |
| 13 | 5.0 |
| 14 | 5.0 |
| 15 | 5.0 |
| 16 | 5.0 |
| 17 | 5.0 |
| 18 | 5.0 |
| 19 | 5.5 |
| 20 | 5.0 |
| 21 | 5.0 |
| 22 | 5.3 |
| 23 | 5.4 |

TABLE I (cont'd)

| Example No. | Agonist Testing (mean pD2) |
|---|---|
| 24 | 5.5 |
| 25 | 5.8 |
| 27 | 6.3 |
| 28 | 5.6 |
| 29 | 5.5 |
| 30 | 5.0 |
| 31 | 5.0 |
| 32 | 5.6 |
| 33 | 5.0 |
| 34 | 5.2 |
| 35 | 5.0 |
| 36 | 5.0 |
| 37 | 5.0 |
| 38 | 5.5 |
| 39 | 5.0 |
| 40 | 6.5 |
| 41 | 6.6 |
| 42 | 5.0 |
| 43 | 5.0 |
| 44 | 5.0 |
| 45 | 5.0 |
| 46 | 5.0 |
| 47 | 5.5 |
| 48 | 5.0 |
| 49 | 5.0 |
| 50 | 5.0 |
| 51 | 5.0 |
| 52 | 6.6 |
| 53 | 5.0 |
| 54 | 6.2 |
| 55 | 5.9 |
| 56 | 6.2 |
| 57 | 7.1 |
| 58 | 6.1 |
| 59 | 5.0 |
| 60 | 6.2 |
| 61 | 6.1 |
| 62 | 6.0 |
| 63 | 5.0 |
| 64 | 5.0 |
| 65 | 5.0 |

# CLAIMS

1. A compound of the formula:

S - S

$R_1-R_2-R_3-(R_4)_m-R_5-(R_6)_n-R_7-R_8-R_9-R_{10}-R_{11}-R_{12}$

wherein $R_1$ is absent, hydrogen, acetyl, Ser, SerSer, D-Arg, Arg, Apa, Ahepa, Aoa, ArgSerSer or ArgArgSerSer; $R_2$ is Cys, Mpa or Pen; $R_3$ is Phe Cha, L-Tic, D-Tic or Nal; $R_4$ is Gly, Pro, D-Ala or Ala; $R_5$ is Gly, Pro, Aca, mAMBA, Ala or D-Ala; $R_6$ is Arg, D-Arg, Lys, or Gly; $R_7$ is Ile, Leu, PrGly or Gly; $R_8$ is Asp or Gly; $R_9$ is D-Arg, Arg, Lys or Gly; $R_{10}$ is Ile; $R_{11}$ is Cys or Pen; $R_{12}$ is OH, $NH_2$, ChaArg, Arg, ProArg, GlyArg, PheArg PheLys, AoaArg, AsnSer, SerPheArg or AsnSerPheArg; m is 0 or 1 and n is 0 or 1; or pharmaceutically acceptable salts, esters or amides thereof.

2. The compound of Claim 1 wherein $R_1$ is ArgArgSerSer; $R_2$ is Cys; $R_3$ is Phe; $R_4$ and $R_5$ are each Gly; $R_6$ is Arg; $R_7$ is Ile; $R_8$ is Asp; $R_9$ is Arg; $R_{10}$ is Ile; $R_{11}$ is Cys; $R_{12}$ is PheArg; and m and n are each 1.

3. The compound of Claim 1 wherein $R_1$ is SerSer; $R_2$ is Cys; $R_3$ is Phe; $R_4$ and $R_5$ are each Gly; $R_6$ is Arg; $R_7$ is Ile; $R_8$ is Asp; $R_9$ is Arg; $R_{10}$ is Ile; $R_{11}$ is Cys; $R_{12}$ is PheArg; and m and n are each 1.

4. The compound of Claim 1 wherein $R_1$ is SerSer; $R_2$ is Pen; $R_3$ is Phe; $R_4$ and $R_5$ are each Gly; $R_6$ is Arg; $R_7$ is Ile; $R_8$ is Asp; $R_9$ is Arg;

$R_{10}$ is Ile; $R_{11}$ is Cys; $R_{12}$ is PheArg; and m and n are each 1.

5. A compound of the formula:

S – S

$$R_1-R_2-R_3-(R_4)_m-R_5-(R_6)_n-R_7-R_8-R_9-R_{10}-R_{11}-R_{12}$$

wherein $R_1$ is hydrogen, Aoa, Aha, Apa, Arg, D-Arg, SerSer, or ArgArgSerSer; $R_2$ is Cys or Pen; $R_3$ is Phe, Cha, L-Tic, D-Tic or L-beta-Nal; $R_4$ is Gly; $R_5$ is Gly or mAMBA; $R_6$ is Arg, Lys or D-Arg; $R_7$ is Ile, Leu or PrGly; $R_8$ is Asp; $R_9$ is Arg or D-Arg; $R_{10}$ is Ile; $R_{11}$ is Cys; $R_{12}$ is OH, $NH_2$, AsnSer, AsnSerPhe, AsnSerPheArg, PheArg, Arg, AoaArg, L-TicArg, D-TicArg or ChaArg; or pharmaceutically acceptable salts, esters or amides thereof.

6. A composition for treating hypertension comprising a therapeutically effective amount of a compound of Claim 1 and a carrier.

7. Use of a compound of claim 1 in preparing a drug for treating hypertension.

MODIANO, JOSIF, PISANTY & STAUB
EUROPEAN PATENT ATTORNEYS - U.S. PATENT AGENTS
BAADERSTR. 3 - D-8000 MÜNCHEN 5 (WEST GERMANY) - PHONE (89) 221.216
FROM USA: PHONE (01392) 879.842 OR (014989) 221.216 - TELEX 843.334.846
TELEFAX (01392) 863.8… (EMERGENCY TELEFAX MUNICH 014989 - … 29.17.80)

DR. ING. G. MODIANO
DR. ING. A. JOSIF
DR. ING. M. PISANTY*
DR. ING. G. STAUB*
DR. ING. N. ZANOTTI*
DIPL. ING. C. S. RENIERO*
S. L. A. MODIANO*

*EUROP. PATENT ATTORNEYS ONLY

APR 12 1988

EPA · EPO · OEB
MÜNCHEN
Empfang bestätigt
Receipt acknowledged
Accuse reception
SD

EUROPEAN PATENT OFFICE
Erhardtstr. 27
D - 8000 MÜNCHEN 2
GERMANIA OCC.LE

EPA · EPO · OEB
DG 1

Re: European patent application No. 87118060.0
filed on December 7, 1987
in the name of ABBOTT LABORATORIES
Our ref: 49537/PAB/ms.

---

REQUEST FOR CORRECTION UNDER RULE 88 EPC.

Dear Sirs,

it has just come to the Applicant's attention that in the description, which was transformed from the format required by the U.S.P.T.O. into A4 format as required by the EPO, some minor errors of transcription have occured.

Accordingly, the applicant herewith respectfully submits hand-amended description pages 35, 37, 40-43, and 45-47, and requests that originally filed pages 35, 37, 40-43, and 45-47 be substituted thereby, under the provisions of Rule 88 EPC.

It will be noted that the correction is obvious because it makes the respective compound names, reagents and amino acid analysis consistent and thus it is immediately evident that nothing else would have been intended than what is offered as the correction. Therefore, it is believed that the correction is allowable under Rule 88 EPC.

Favourable action is respectfully solicited.

Respectfully submitted,

Dr. G. Modiano

Professional Representative for the Applicant.

Enclosures: Hand amended description pages 35, 37, 40-43 and 45-47.